# EUROPÄISCHE PATENTSCHRIFT

(11) **EP 3 542 760 B1**
(45) Veröffentlichungstag und Bekanntmachung des Hinweises auf die Patenterteilung: **16.06.2021**
(21) Anmeldenummer: 19163783.4
(22) Anmeldetag: 19.03.2019
(51) Int. Cl.: A61F 2/32, A61F 2/46, A61F 2/38, A61F 2/30

(54) **KNIESPACERSYSTEM MIT SPÜLVORRICHTUNG**
KNEE SPACER SYSTEM WITH FLUSHING DEVICE
SYSTÈME D'ESPACEUR DU GENOU POURVU DE DISPOSITIF DE RINÇAGE

(30) Priorität: 21.03.2018 DE 102018106704
(43) Veröffentlichungstag der Anmeldung: 25.09.2019
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- EP-A1- 3 170 476
- US-A1- 2014 343 560
- US-A1- 2017 312 087
- US-A1- 2017 354 507

## Beschreibung

Die Erfindung betrifft ein temporäres Kniespacersystem zum zeitweisen Ersetzen eines Kniegelenks, wie in den Ansprüchen definiert, das für die Interimsphase von zweizeitigen septischen Revisionen von Kniegelenk-Totalendoprothesen bestimmt ist. Das Kniespacersystem kann besonders bei solchen zweizeitigen septischen Revisionen Anwendung finden, bei denen zwei oder mehrere mikrobielle Keime ursächlich für eine Infektion der Kniegelenk-Totalendoprothese und des umgebenden Gewebes sind.

Kniegelenk-Totalendoprothesen werden in großem Umfang weltweit implantiert. Die Standzeit dieser Implantate beträgt heute zirka 15 Jahre. Leider kommt es in einem geringen Prozentsatz vor, dass Kniegelenk-Totalendoprothesen von mikrobiellen Keimen, besonders Gram-positiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Kniegelenk-Totalendoprothesen erreichen. Bei einer Besiedlung von Kniegelenk-Totalendoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Kniegelenk-Totalendoprothesen, die einzeitige septische Revision und die zweizeitige septische Revision. Daneben gibt es noch eine Anzahl weiterer Behandlungsverfahren, wie zum Beispiel die Anwendung von Saug-Spül-Drainagen.

Bei der einzeitigen Revision wird innerhalb einer OP zuerst die infizierte Kniegelenk-Totalendoprothese entfernt, anschließend radikal debridiert und dann wird eine Revisions-Kniegelenkendoprothese implantiert.

Bei zweizeitigen septischen Revisionen wird in einer ersten OP zuerst die infizierte Kniegelenk-Totalendoprothese entfernt, dann wird debridiert und anschließend erfolgt die Implantation eines Kniegelenkspacers. Eine Tibiakomponente und eine Femurkomponente des Kniegelenkspacers werden mit Knochenzement an der Tibia beziehungsweise am Femur verankert. Der Kniegelenkspacer verbleibt bis zu mehreren Wochen im Patienten, bis die Entzündung abgeklungen ist und die klinischen Entzündungsmarker zurückgegangen sind. Dann wird in einer zweiten OP der Kniegelenkspacer entfernt und nach erneutem Debridement eine Revisions-Kniegelenkendoprothese implantiert.

Es ist bekannt, mit Antibiotika ausgerüstete Spacer zu verwenden. Diese Spacer können einerseits vom OP-Personal während der OP selbst aus PMMA-Knochenzementpulver, Antibiotika und Monomerflüssigkeit hergestellt werden und andererseits ist es auch üblich, industriell aus Knochenzement vorgefertigte Spacer einzusetzen.

Bei den bisherigen Spacern werden dem Zementpulver vor der eigentlichen Herstellung des Spacers Antibiotika zugesetzt. Mit diesem antibiotisch modifizierten Knochenzementpulver werden anschließend die Spacer gegossen, die dann durch Polymerisation mit Hilfe einer dem Zementpulver zugesetzten Monomerflüssigkeit aushärten. Der Knochenzementteig schließt dabei die Antibiotika im Wesentlichen ein. Nur die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab. Danach werden nur noch geringe Mengen der Antibiotika freigesetzt. Die Hauptmenge der zugesetzten Antibiotika verbleibt im ausgehärteten Knochenzement der Spacer. Eine nachträgliche Veränderung der Art und der Anzahl der eingesetzten Antibiotika ist nach der Herstellung des Spacers beziehungsweise nach der Implantation bei den bisherigen aus Knochenzement gefertigten Spacern nicht möglich. Weiterhin ist die Einstellung einer definierten Konzentration an antimikrobiellen Wirkstoffen im Wundsekret beziehungsweise der die Spacerkomponenten umgebenden Körperflüssigkeit ebenfalls nicht möglich.

Industriell vorgefertigte Kniegelenkspacer, die ein oder zwei Antibiotika enthalten, sind aus einer Reihe von Dokumenten bekannt. Exemplarisch dafür seien die Patentanmeldung WO 2017/199 131 A1 und die Patente EP 2 758 004 B1, US 8 562 687 B2, EP 2 781 206 B1 und EP 2 826 445 B1 genannt.

Im Patent EP 1 971 293 B1 wird ein Kniegelenkspacer vorgeschlagen, der ein Reservoir für Wirkstofflösungen mit einem Pumpmechanismus enthält. Der Pumpmechanismus wird durch die Laufbewegung des Patienten betätigt und pumpt die Wirkstofflösung aus dem Reservoir. Es handelt sich dabei im Prinzip um eine Membranpumpe.

Im Patent EP 2 740 468 B1 wird ein Implantat offenbart, das Kapillaren enthält, die mit Wirkstofflösungen befüllt werden können. Weiterhin enthält das Implantat das Metall Magnesium an einem Ende der Kapillaren. In Gegenwart von Feuchtigkeit korrodiert das Magnesium. Es zersetzt sich bei Einwirkung von Wasser unter Freisetzung von Wasserstoff. Dieses Gas treibt die Wirkstofflösung kontinuierlich über einen Zeitraum von mehreren Tagen aus den Kapillaren.

Die US 2017/0354507 A1 offenbart einen Kniespacer, der in seinem Inneren ein Flüssigkeitsreservoir enthält. Aus der EP 3 170 476 A1 ist ein Implantat zur Abgabe von Flüssigkeiten bekannt.

Die US 2018/0008423 A1 beschreibt einen Kniegelenkspacer, der bei infizierten Kniegelenk-Totalendoprothesen eingesetzt werden kann. Im Gegensatz zu einzeitigen und zweizeitigen septischen Revisionen verbleiben von der infizierten Kniegelenk-Totalendoprothese die Femurkomponente und der Tray der Kniekomponente im Patienten. Es wird nur das Kunststoffinlay entfernt und durch den Spacer ersetzt. Der Spacer bildet dazu das Kunststoffinlay nach. Das hat den Nachteil, dass Keime, die sich an anderen Teilen der Kniegelenk-Totalendoprothese festgesetzt haben, nicht oder nur schlecht von dem Wirkstoff aus dem Spacer erreicht werden können. Der Spacer ist dadurch charakterisiert, dass er ein Reservoir zur Aufnahme von antibiotischen Lösungen enthält. Die Abgabe der antibiotischen Lösungen erfolgt durch proximale Öffnungen in der Lauffläche des Spacers. Das Reservoir des Spacers kann über einen Zugang neu befüllt werden. Dazu ist der Zugang mit einem Port ausgerüstet. Problematisch ist bei derartigen Spacern, dass Flüssigkeit in das Reservoir eingebracht werden muss, ohne dass dabei ein Druckausgleich erfolgen kann. Das bedeutet, dass die antibiotische Flüssigkeit bei großen Flüssigkeitsvolumina und hohem Druck bei der Injektion unter Umständen in das Weichgewebe beziehungsweise in ableitende Blutgefäße gepresst wird. Eine Ableitung von Wundsekret und Debris kann bei diesem Spacer nicht erfolgen.

Bei der zweizeitigen septischen Revision werden bei der Implantation der Kniegelenkspacer auch Drainagen eingesetzt, die zur Ableitung von Wundsekret, Blut und Debris bestimmt sind. Die Drainagen verbleiben bis zu mehreren Tagen im Patienten. Die von den Spacerkomponenten freigesetzten antibiotischen Wirkstoffe werden vom Wundsekret aufgenommen und über die Drainage nach außen abgeführt. Dadurch geht ein Teil der antimikrobiellen Wirkstoffe zum Schutz der Spaceroberfläche vor mikrobieller Besiedlung verloren.

Es wurde im Rahmen der vorliegenden Erfindung erkannt, dass es wünschenswert wäre, wenn die Spaceroberfläche von einer antimikrobiellen Wirkstofflösung umgeben wäre, deren Wirkstoffkonzentration exakt eingestellt werden kann und deren Konzentration über mehrere Tage unabhängig vom Wundsekretstrom erhalten bleiben würde. Weiterhin wäre es wünschenswert, dass man die Art und die Anzahl der mikrobiellen Wirkstoffe auch nach der Implantation des Kniegelenkspacers variieren kann, um zum Beispiel auf erst später nachgewiesene mikrobielle Keime reagieren zu können. Gleichzeitig soll der Patient das Kniegelenk bewegen können, um eine Verkürzung der Sehnen und der Muskulatur und eine Degeneration der Beinmuskulatur zu verhindern und so die Rehabilitation zu verkürzen.

Die Aufgabe der vorliegenden Erfindung besteht darin, die Nachteile des Stands der Technik zu überwinden. Insbesondere besteht die Aufgabe der Erfindung in der Entwicklung eines temporären Kniespacersystems, das verbesserte und patientenspezifischere Anwendungen ermöglicht. Es soll vorzugsweise ein Kniespacersystem entwickelt werden, mit dem eine medizinische Spülflüssigkeit gezielt im Bereich des Knies eingesetzt werden kann. Gleichzeitig soll das Kniespacersystem eine Beweglichkeit des Knies im beim Patienten eingesetzten Zustand ermöglichen.

Die Aufgabe der Erfindung ist es dabei insbesondere, ein artikulierendes Kniespacersystem zu entwickeln, das für die Interimsphase von zweizeitigen septischen Revisionen von Kniegelenk-Totalendoprothesen bestimmt ist. Das Kniespacersystem soll den Raum nach der Entfernung der Kniegelenk-Totalendoprothesen und dem nachfolgenden Debridement so ausfüllen, dass eine Rückbildung des Bandapparates und der Muskulatur verhindert wird. Das zu entwickelnde Kniespacersystem soll es ermöglichen, dass die artikulierenden Spaceroberflächen, das die Spacerkomponenten umgebende Weichgewebe und zumindest ein Teil des umgebenden Knochengewebes mit einer antiseptischen oder antibiotischen Spülflüssigkeiten kontinuierlich oder auch diskontinuierlich gespült werden können. Weiterhin soll das Kniespacersystem möglichst so gestaltet werden, dass die Spülflüssigkeit nicht unkontrolliert den Raum um das Kniespacersystem verlassen kann. Das Kniespacersystem soll mit dem Knochengewebe der Tibia und des Femurs in der Weise mit Knochenzement verbunden werden können, dass der Austritt der Spülflüssigkeit aus dem Kniespacersystem und auch die Aufnahme der Spülflüssigkeit in das Kniespacersystem zur Ableitung nicht gestört oder unterbrochen wird. Das Kniespacersystem soll ferner möglichst so beschaffen sein, dass nach einer Beendigung der Spülungen mit der medizinischen Spülflüssigkeit die Spülflüssigkeitszuführung und die Spülflüssigkeitsableitung entfernt werden können, ohne dass sich eine Beeinträchtigung der Artikulation der Spacerkomponenten ergibt.

Die Aufgaben der Erfindung werden gelöst durch ein temporäres Kniespacersystem zum zeitweisen Ersetzen eines Kniegelenks, das Kniespacersystem aufweisend
A) eine Tibiakomponente, wobei die Tibiakomponente einen Tibia-Prothesenkörper aufweist, wobei der Tibia-Prothesenkörper zwei Laufflächen auf einer proximalen Seite der Tibiakomponente aufweist und zumindest eine Befestigungsfläche zur Befestigung der Tibiakomponente an einer Tibia auf einer distalen Seite des Tibia-Prothesenkörpers aufweist,
B) ein erstes röhrenförmiges und flüssigkeitsdurchlässiges Verbindungsmittel zum Zuführen einer medizinischen Spülflüssigkeit in den Tibia-Prothesenkörper,
C) ein zweites röhrenförmiges und flüssigkeitsdurchlässiges Verbindungsmittel zum Abführen der Spülflüssigkeit aus dem Tibia-Prothesenkörper,
D) eine Spülflüssigkeits-Einlassöffnung in einer Oberfläche des Tibia-Prothesenkörpers, wobei das erste Verbindungsmittel mit der Spülflüssigkeits-Einlassöffnung flüssigkeitsdurchlässig verbunden oder verbindbar ist,
E) eine Spülflüssigkeits-Auslassöffnung in der Oberfläche des Tibia-Prothesenkörpers, wobei das zweite Verbindungsmittel mit der Spülflüssigkeits-Auslassöffnung flüssigkeitsdurchlässig verbunden oder verbindbar ist,
F) zumindest eine Spülflüssigkeitsaustrittsöffnung in der Oberfläche des Tibia-Prothesenkörpers, die im Inneren des Tibia-Prothesenkörpers mit der Spülflüssigkeits-Einlassöffnung flüssigkeitsdurchlässig verbunden ist, und
G) zumindest eine Spülflüssigkeitseintrittsöffnung in der Oberfläche des Tibia-Prothesenkörpers, die im Inneren des Prothesenkörpers mit der Spülflüssigkeits-Auslassöffnung flüssigkeitsdurchlässig verbunden ist, wobei
die zumindest eine Spülflüssigkeitsaustrittsöffnung und die zumindest eine Spülflüssigkeitseintrittsöffnung außerhalb der zumindest einen Befestigungsfläche angeordnet sind und wobei die zumindest eine Spülflüssigkeitsaustrittsöffnung im Inneren des Prothesenkörpers nicht flüssigkeitsdurchlässig mit der Spülflüssigkeits-Auslassöffnung verbunden ist und die zumindest eine Spülflüssigkeitseintrittsöffnung im Inneren des Prothesenkörpers nicht flüssigkeitsdurchlässig mit der Spülflüssigkeits-Einlassöffnung verbunden ist.

Das erfindungsgemäße Kniespacersystem kann also nur eine Tibiakomponente aufweisen und muss keine weiteren Teile und also auch keine Femurkomponente aufweisen. Bevorzugt weist das Kniespacersystem jedoch zusätzlich auch eine Femurkomponente auf.

Die Spülflüssigkeit kann theoretisch auch zunächst durch das erste Verbindungsmittel eingeleitet werden und durch das zweite Verbindungsmittel abgeführt werden und anschließend durch das zweite Verbindungsmittel eingeleitet werden und durch das erste Verbindungsmittel abgeführt werden. Die Prothese wird dann alternierend betrieben. Es wird jedoch erfindungsgemäß bevorzugt, dass die Prothese nur in einer Fließrichtung der Spülflüssigkeit betrieben wird.

Vorzugsweise ist das erste röhrenförmige flüssigkeitsdurchlässige Verbindungsmittel ein Schlauch mit einem Adapter oder einem Anschluss und das zweite röhrenförmige flüssigkeitsdurchlässige Verbindungsmittel ebenfalls ein Schlauch mit einem Adapter oder einem Anschluss.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Kniespacersystem so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Bevorzugt kann vorgesehen sein, dass das Kniespacersystem zur Anwendung eines antibiotischen Wirkstoffs geeignet ist, der eine Polymerisation oder eine radikalische Polymerisation von PMMA verhindert oder beeinträchtigt. Insbesondere kann vorgesehen sein, dass das Kniespacersystem zur Anwendung von Rifampicin und Metronidazol geeignet ist.

Es kann erfindungsgemäß auch vorgesehen sein, dass die zumindest eine Befestigungsfläche begrenzt ist oder dass die zumindest eine Befestigungsfläche mit einem umlaufenden und sich aus der Oberfläche des Tibia-Prothesenkörpers erhebenden Steg begrenzt ist, so dass die zumindest eine Befestigungsfläche zur Aufnahme von Knochenzementteig innerhalb des Stegs geeignet ist.

Hierdurch kann ein begrenzter und dadurch ein bestimmter Bereich zur Befestigung der Tibiakomponente verwendet werden. Bei korrekter Anwendung des Kniespacersystems kann dadurch verhindert werden, dass das erste und das zweite Verbindungsmittel sowie die Spülflüssigkeits-Einlassöffnung und die Spülflüssigkeits-Auslassöffnung mit Knochenzement bedeckt werden und dadurch in ihrer Funktion beeinträchtigt werden. Insbesondere kann verhindert werden, dass der ausgehärtete Knochenzement ein Abziehen beziehungsweise ein Lösen des ersten und des zweiten Verbindungsmittels vom Tibia-Prothesenkörper verhindert.

Es ist vorgesehen, dass die zumindest eine Spülflüssigkeitsaustrittsöffnung im Inneren des Prothesenkörpers nicht flüssigkeitsdurchlässig mit der Spülflüssigkeits-Auslassöffnung verbunden ist und die zumindest eine Spülflüssigkeitseintrittsöffnung im Inneren des Prothesenkörpers nicht flüssigkeitsdurchlässig mit der Spülflüssigkeits-Einlassöffnung verbunden ist.

Hierdurch wird sichergestellt, dass mit dem Kniespacersystem ein Kreislauf mit der Spülflüssigkeit erzeugt werden kann, ohne dass die Spülflüssigkeit zunächst eingedrückt und anschließend abgesaugt werden muss. Dies ist in der Anwendung schonender. Das Kniespacersystem kann alternativ aber auch so verwendet werden, dass die Spülflüssigkeit zunächst in das Kniespacersystem eingeleitet wird und durch die zumindest eine Spülflüssigkeitsaustrittsöffnung und die zumindest eine Spülflüssigkeitseintrittsöffnung austritt und anschließend die Spülflüssigkeit wieder abgesaugt wird und dabei durch die zumindest eine Spülflüssigkeitsaustrittsöffnung und die zumindest eine Spülflüssigkeitseintrittsöffnung wieder in den Tibia-Prothesenkörper eingesaugt wird. Bevorzugt wird jedoch ein Kreislauf mit der Spülflüssigkeit erzeugt.

Gemäß einer besonders bevorzugten Weiterbildung der vorliegenden Erfindung kann vorgesehen sein, dass die Tibiakomponente wenigstens zwei Spülflüssigkeitsaustrittsöffnungen und wenigstens zwei Spülflüssigkeitseintrittsöffnungen in der Oberfläche des Tibia-Prothesenkörpers aufweist, die an unterschiedlichen Seiten des Tibia-Prothesenkörpers angeordnet sind, wobei vorzugsweise eine Spülflüssigkeitsaustrittsöffnung und eine Spülflüssigkeitseintrittsöffnung auf der proximalen Seite des Tibia-Prothesenkörpers angeordnet sind und eine Spülflüssigkeitsaustrittsöffnung und eine Spülflüssigkeitseintrittsöffnung auf der distalen Seite des Tibia-Prothesenkörpers angeordnet sind.

Hiermit können zwei separate Kreisläufe mit der medizinischen Spülflüssigkeit erzeugt werden, die sowohl auf der distalen als auch auf der proximalen Seite der eingesetzten Tibiakomponente verlaufen.

Ferner kann vorgesehen sein, dass die zumindest eine Spülflüssigkeitsaustrittsöffnung und die zumindest eine Spülflüssigkeitseintrittsöffnung voneinander paarweise beabstandet sind, wobei der Abstand zwischen jedem Paar der zumindest einen Spülflüssigkeitsaustrittsöffnung und der zumindest einen Spülflüssigkeitseintrittsöffnung zumindest 5 mm, bevorzugt zumindest 20 mm und besonders bevorzugt zumindest 30 mm beträgt.

Hierdurch wird sichergestellt, dass der Kreislauf der medizinischen Spülflüssigkeit eine größere Strecke (zumindest 5 mm) an der äußeren Oberfläche des Tibia-Prothesenkörpers zurücklegen muss und dort zum Spülen zur Verfügung steht.

Des Weiteren kann vorgesehen sein, dass das erste Verbindungsmittel an der zur Verbindung mit der Spülflüssigkeits-Einlassöffnung abgewandten Seite und das zweite Verbindungmittel an der zur Verbindung mit der Spülflüssigkeits-Auslassöffnung abgewandten Seite jeweils einen Adapter aufweist, insbesondere jeweils ein Luer-Lock-Adapter aufweist.

Hierdurch kann das Kniespacersystem bequem an ein medizinisches Spülflüssigkeitsreservoir mit einer Pumpe und an einen Auffangbehälter zum Aufnahmen der gebrauchten Spülflüssigkeit angeschlossen werden.

Bevorzugt kann auch vorgesehen sein, dass an der Spülflüssigkeits-Einlassöffnung im Inneren des Tibia-Prothesenkörpers oder an der Oberfläche des Tibia-Prothesenkörpers eine selbstdichtende Kupplung angeordnet ist und an der Spülflüssigkeits-Auslassöffnung im Inneren des Tibia-Prothesenkörpers oder an der Oberfläche des Tibia-Prothesenkörpers eine selbstdichtende Kupplung angeordnet ist, wobei das erste Verbindungsmittel lösbar mit der Spülflüssigkeits-Einlassöffnung verbunden oder verbindbar ist und das zweite Verbindungsmittel lösbar mit der Spülflüssigkeits-Auslassöffnung verbunden oder verbindbar ist.

Hierdurch verschließen sich die Spülflüssigkeits-Einlassöffnung und die Spülflüssigkeits-Auslassöffnung oder die Flüssigkeitsleitungen dahinter selbsttätig, wenn das erste Verbindungsmittel beziehungsweise das zweite Verbindungsmittel abgezogen beziehungsweise getrennt wird. Dadurch wird der Durchgang verschlossen, wenn keine Spülflüssigkeit mehr durch das Kniespacersystem geleitet werden soll.

Besonders variable Kniespacersysteme können sich dadurch auszeichnen, dass die Spülflüssigkeits-Einlassöffnung eine erste Spülflüssigkeits-Einlassöffnung ist und die Spülflüssigkeits-Auslassöffnung eine erste Spülflüssigkeits-Auslassöffnung ist, wobei zusätzlich eine zweite Spülflüssigkeits-Einlassöffnung und eine zweite Spülflüssigkeits-Auslassöffnung in der Oberfläche des Tibia-Prothesenkörpers vorgesehen sind, wobei an der ersten Spülflüssigkeits-Einlassöffnung, der zweiten Spülflüssigkeits-Einlassöffnung, der ersten Spülflüssigkeits-Auslassöffnung und der zweiten Spülflüssigkeits-Auslassöffnung jeweils eine selbstdichtende Kupplung angeordnet ist, wobei das erste Verbindungsmittel flüssigkeitsdicht und lösbar mit der ersten Spülflüssigkeits-Einlassöffnung und mit der zweiten Spülflüssigkeits-Einlassöffnung verbindbar ist und das zweite Verbindungsmittel flüssigkeitsdicht und lösbar mit der ersten Spülflüssigkeits-Auslassöffnung und mit der zweiten Spülflüssigkeits-Auslassöffnung verbindbar ist, wobei die erste Spülflüssigkeits-Einlassöffnung und die zweite Spülflüssigkeits-Einlassöffnung im Tibia-Prothesenkörper miteinander flüssigkeitsdurchlässig verbunden sind und die erste Spülflüssigkeits-Auslassöffnung und die zweite Spülflüssigkeits-Auslassöffnung im Tibia-Prothesenkörper miteinander flüssigkeitsdurchlässig verbunden sind.

Durch diese Maßnahmen kann der Zugang zum Kniespacersystem variabel gewählt werden und an die jeweilige Situation individuell und patientenspezifisch angepasst werden. Die selbstdichtende Kupplung verschließt sich selbsttätig, wenn kein Verbindungsmittel (das erste oder das zweite Verbindungsmittel) angeschlossen ist oder ein Verbindungsmittel entfernt wird und öffnet sich, wenn ein Verbindungsmittel angeschlossen wird, und bleibt offen solange das Verbindungsmittel angeschlossen ist.

Bei Kniespacersystemen mit mehreren Spülflüssigkeits-Einlassöffnungen und Spülflüssigkeits-Auslassöffnungen kann vorgesehen sein, dass die erste Spülflüssigkeits-Einlassöffnung und die erste Spülflüssigkeits-Auslassöffnung auf einer ersten Seite der Tibiakomponente bezüglich der Sagittalebene angeordnet ist und die zweite Spülflüssigkeits-Einlassöffnung und die zweite Spülflüssigkeits-Auslassöffnung auf einer zweiten Seite der Tibiakomponente bezüglich der Sagittalebene angeordnet ist, wobei besonders bevorzugt, die erste Spülflüssigkeits-Einlassöffnung und die erste Spülflüssigkeits-Auslassöffnung bezüglich der Sagittalebene spiegelbildlich zur zweiten Spülflüssigkeits-Einlassöffnung und zur zweiten Spülflüssigkeits-Auslassöffnung in der Oberfläche des Tibia-Prothesenkörpers angeordnet sind. Hierdurch lassen sich die Anschlüsse auf anatomisch besonders günstige Weise belegen.

Es kann auch vorgesehen sein, dass die Querschnittsfläche der zumindest einen Spülflüssigkeitseintrittsöffnung zumindest genauso groß ist wie die Querschnittsfläche der Spülflüssigkeits-Einlassöffnung und/oder die Summe der Querschnittsflächen der zumindest einen Spülflüssigkeitsaustrittsöffnung zumindest genauso groß ist wie die Querschnittsfläche der Spülflüssigkeits-Auslassöffnung.

Hierdurch kann ein Staudruck im Inneren des Tibia-Prothesenkörpers vermieden werden.

Des Weiteren kann vorgesehen sein, dass die zumindest eine Spülflüssigkeitsaustrittsöffnung und die zumindest eine Spülflüssigkeitseintrittsöffnung durch einen umlaufenden und sich aus der Oberfläche des Prothesenkörpers erhebenden Steg von der zumindest einen Befestigungsfläche abgegrenzt sind.

Bei korrekter Anwendung des Kniespacersystems kann dadurch verhindert werden, dass das erste und das zweite Verbindungsmittel sowie die Spülflüssigkeits-Einlassöffnung und die Spülflüssigkeits-Auslassöffnung mit Knochenzement bedeckt werden und dadurch in ihrer Funktion beeinträchtigt werden. Insbesondere kann verhindert werden, dass der ausgehärtete Knochenzement ein Abziehen beziehungsweise ein Lösen des ersten und des zweiten Verbindungsmittels vom Tibia-Prothesenkörper verhindert.

Es kann ferner vorgesehen sein, dass wenigstens eine Spülflüssigkeitsaustrittsöffnung der zumindest einen Spülflüssigkeitsaustrittsöffnung und/oder wenigstens eine Spülflüssigkeitseintrittsöffnung der zumindest einen Spülflüssigkeitseintrittsöffnung zwischen den Laufflächen angeordnet ist oder sind.

Hiermit wird erreicht, dass die medizinische Spülflüssigkeit zwischen den Laufflächen und dicht bei einer das Gegenstück zur Tibiakomponente bildenden Femurkomponente austritt, insbesondere zwischen den Laufflächen und dicht bei Kondylen der Femurkomponente austritt, und dadurch die Spülflüssigkeit bei einer Bewegung des Knies zwischen der Tibiakomponente und der Femurkomponente verteilt wird. Dadurch kann das Gelenk bei einer Bewegung des Kniespacersystems gut mit der medizinischen Spülflüssigkeit gespült werden.

Gemäß einer Weiterentwicklung kann vorgesehen sein, dass die zumindest eine Spülflüssigkeitsaustrittsöffnung und die zumindest eine Spülflüssigkeitseintrittsöffnung außerhalb den Laufflächen angeordnet sind, wobei vorzugsweise eine Spülflüssigkeitsaustrittsöffnung der zumindest einen Spülflüssigkeitsaustrittsöffnung und eine Spülflüssigkeitseintrittsöffnung der zumindest einen Spülflüssigkeitseintrittsöffnung neben einer der Laufflächen angeordnet sind, besonders bevorzugt innerhalb von 3 mm neben einer der Laufflächen angeordnet sind.

Hiermit wird sichergestellt, dass die zumindest eine Spülflüssigkeitsaustrittsöffnung und die zumindest eine Spülflüssigkeitseintrittsöffnung nicht die Funktion der Laufflächen beeinträchtigen. Zudem kann so eine mechanische Belastung und ein damit einhergehender unerwünschter Abrieb der Kanten der zumindest einen Spülflüssigkeitsaustrittsöffnung und der zumindest einen Spülflüssigkeitseintrittsöffnung verhindert werden.

Zur Steuerung des Spülflüssigkeitskreislaufs kann vorgesehen sein, dass in dem ersten Verbindungsmittel oder in der Spülflüssigkeits-Einlassöffnung ein erstes Ventilelement angeordnet ist, das ein Rückfließen der Spülflüssigkeit in das erste Verbindungsmittel verhindert, und/oder in dem zweiten Verbindungsmittel oder in der Spülflüssigkeits-Auslassöffnung ein zweites Ventilelement angeordnet ist, das ein Rückfließen der Spülflüssigkeit in das zweite Verbindungsmittel verhindert, wobei bevorzugt das erste und das zweite Ventilelement ausgewählt sind aus einem Rückschlagventil, einem Kugelventil mit Feder, einem Lippenventil, einem Bunsenventil oder einem Plattenventil.

Dadurch kann ein umlaufender Kreislauf der medizinischen Spülflüssigkeit erzwungen werden. Zudem kann so ein Rücklaufen der benutzten medizinischen Spülflüssigkeit und damit eine Kontamination der Spülflüssigkeitsquelle beziehungsweise von Leitungen im Tibia-Prothesenkörper verhindert werden.

Des Weiteren kann hierzu vorgesehen sein, dass in einer ersten Leitung innerhalb des Tibia-Prothesenkörpers, die die zumindest eine Spülflüssigkeitseintrittsöffnung mit der Spülflüssigkeits-Auslassöffnung flüssigkeitsdurchlässig verbindet, ein erstes Ventil angeordnet ist, das nur durch Anlegen eines Unterdrucks an der Spülflüssigkeits-Auslassöffnung zu öffnen ist und dass ein Zurückfließen der Spülflüssigkeit in die erste Leitung verhindert, und/oder in einer zweiten Leitung innerhalb des Tibia-Prothesenkörpers, die die zumindest eine Spülflüssigkeitsaustrittsöffnung mit der Spülflüssigkeits-Einlassöffnung flüssigkeitsdurchlässig verbindet, ein zweites Ventil angeordnet ist, das nur durch Anlegen eines Unterdrucks an der Spülflüssigkeits-Einlassöffnung zu öffnen ist und dass ein Zurückfließen der Spülflüssigkeit in die zweite Leitung verhindert.

Auch dadurch kann ein Rücklaufen der benutzten medizinischen Spülflüssigkeit verhindert werden. Zudem kann so sichergestellt werden, dass ohne die Verbindungsmittel noch ein Austausch enthaltener Spülflüssigkeit mit umgebenden Flüssigkeiten stattfindet.

Ferner kann vorgesehen sein, dass in dem Tibia-Prothesenkörper die Spülflüssigkeits-Einlassöffnung, die Spülflüssigkeits-Auslassöffnung, die zumindest eine Spülflüssigkeitsaustrittsöffnung und die zumindest eine Spülflüssigkeitseintrittsöffnung und die flüssigkeitsdurchlässigen Verbindungen ausgebildet sind, wobei der Tibia-Prothesenkörper vorzugsweise aus Kunststoff, Metall, Keramik, Glaskeramik, Knochenzement oder einer Kombination daraus gefertigt ist.

Hierdurch wird ein kompakter Aufbau erreicht und der Tibia-Prothesenkörper gleicht äußerlich bis auf die Öffnungen einer herkömmlichen Tibiakomponente.

Zur besseren Zugänglichkeit der Anschlüsse kann vorgesehen sein, dass die Spülflüssigkeits-Einlassöffnung und die Spülflüssigkeits-Auslassöffnung in einer seitlichen Oberfläche des Tibia-Prothesenkörpers angeordnet sind, vorzugsweise an einer lateralen seitlichen Oberfläche des Tibia-Prothesenkörpers.

Hierdurch kann eine Zufuhr und Abfuhr der Spülflüssigkeit in das und aus dem Kniespacersystem anatomisch leicht und bequem gelegt werden. An der lateralen seitlichen Oberfläche stören die an die Spülflüssigkeits-Einlassöffnung und die Spülflüssigkeits-Auslassöffnung angeschlossenen Verbindungsmittel beim Laufen besonders wenig.

Es wird mit der Erfindung auch vorgeschlagen, dass zumindest eine erste Spülflüssigkeitsaustrittsöffnung der zumindest einen Spülflüssigkeitsaustrittsöffnung auf der proximalen Seite in der Oberfläche des Tibia-Prothesenkörpers angeordnet ist und zumindest eine zweite Spülflüssigkeitsaustrittsöffnung der zumindest einen Spülflüssigkeitsaustrittsöffnung auf der distalen Seite in der Oberfläche des Tibia-Prothesenkörpers angeordnet ist und zumindest eine erste Spülflüssigkeitseintrittsöffnung der zumindest einen Spülflüssigkeitseintrittsöffnung auf der proximalen Seite in der Oberfläche des Tibia-Prothesenkörpers angeordnet ist und zumindest eine zweite Spülflüssigkeitseintrittsöffnung der zumindest einen Spülflüssigkeitseintrittsöffnung auf der distalen Seite in der Oberfläche des Tibia-Prothesenkörpers angeordnet ist.

Hiermit kann sichergestellt werden, dass beide Seiten des Tibia-Prothesenkörpers mit der medizinischen Spülflüssigkeit gespült werden können.

Bei bevorzugten Kniespacersystemen kann auch vorgesehen sein, dass das Kniespacersystem eine Femurkomponente aufweist, wobei die Femurkomponente auf einer distalen Seite zwei Kondylen aufweist.

Hierdurch wird das Kniespacersystem komplettiert und es wird eine zur Tibiakomponente passende Femurkomponente bereitgestellt, deren Kondylen passend auf den Laufflächen der Tibiakomponente gleiten können.

Es kann dabei vorgesehen sein, dass die Kondylen der Femurkomponente dazu geeignet sind, auf den Laufflächen der Tibiakomponente abzurollen. Dass die Kondylen der Femurkomponente auf den Laufflächen der Tibiakomponente abrollen können, bedeutet, dass die Femurkomponente und die Tibiakomponente sich gegeneinander nach Art eines Kniegelenks bewegen können. Der Kniespacer beziehungsweise das Kniespacersystem ist eine artikulierende Prothese, die ein Kniegelenk einschließlich seiner Funktion ersetzen können soll.

Bevorzugt weist die Femurkomponente auf der proximalen Seite mehrere begrenzte Befestigungsflächen auf, die mit einem umlaufenden und sich aus der proximalen Oberfläche der Femurkomponente erhebenden Steg begrenzt sind oder die durch sich aus der proximalen Oberfläche erhebende Stege voneinander getrennt sind. Innerhalb dieser mit den Stegen der Femurkomponente begrenzten Aufnahmen für den Knochenzementteig wird die Femurkomponente an dem Femur befestigt. Zwischen den Befestigungsflächen kann dann die medizinische Spülflüssigkeit fließen, so dass auch die distale Oberfläche des Femurs von der Spülflüssigkeit erreicht werden kann. Zudem können die Stege zur Einstellung der Dicke eines gewünschten Klebespalts verwendet werden.

Bevorzugt kann auch vorgesehen sein, dass die Femurkomponente einen Femur-Prothesenkörper aufweist, wobei der Femur-Prothesenkörper zumindest eine Befestigungsfläche zur Befestigung der Femurkomponente mit einem Knochenzement an einem Femur auf einer proximalen Seite des Femur-Prothesenkörpers aufweist, die Femurkomponente ferner aufweisend ein drittes röhrenförmiges und flüssigkeitsdurchlässiges Verbindungsmittel zum Zuführen einer medizinischen Spülflüssigkeit in den Femur-Prothesenkörper, ein viertes röhrenförmiges und flüssigkeitsdurchlässiges Verbindungsmittel zum Abführen der Spülflüssigkeit aus dem Femur-Prothesenkörper, eine Spülflüssigkeits-Einlassöffnung in einer Oberfläche des Femur-Prothesenkörpers, wobei das dritte Verbindungsmittel mit der Spülflüssigkeits-Einlassöffnung des Femur-Prothesenkörpers flüssigkeitsdurchlässig verbunden oder verbindbar ist, eine Spülflüssigkeits-Auslassöffnung in der Oberfläche des Femur-Prothesenkörpers, wobei das vierte Verbindungsmittel mit der Spülflüssigkeits-Auslassöffnung des Femur-Prothesenkörpers flüssigkeitsdurchlässig verbunden oder verbindbar ist, zumindest eine Spülflüssigkeitsaustrittsöffnung in der Oberfläche des Femur-Prothesenkörpers, die im Inneren des Femur-Prothesenkörpers mit der Spülflüssigkeits-Einlassöffnung des Femur-Prothesenkörpers flüssigkeitsdurchlässig verbunden ist, und zumindest eine Spülflüssigkeitseintrittsöffnung in der Oberfläche des Femur-Prothesenkörpers, die im Inneren des Femur-Prothesenkörpers mit der Spülflüssigkeits-Auslassöffnung des Femur-Prothesenkörpers flüssigkeitsdurchlässig verbunden ist, wobei die zumindest eine Spülflüssigkeitsaustrittsöffnung des Femur-Prothesenkörpers und die zumindest eine Spülflüssigkeitseintrittsöffnung des Femur-Prothesenkörpers außerhalb der zumindest einen Befestigungsfläche des Femur-Prothesenkörpers angeordnet sind.

Hierdurch kann ein Spülen mit der medizinischen Spülflüssigkeit auch im Bereich des Femurs erreicht werden. Eine Verbindung der Kreisläufe der Spülflüssigkeit ist nicht vorgesehen aber grundsätzlich möglich. Beispielsweise könnte das zweite Verbindungsmittel als das dritte Verbindungsmittel verwendet werden, beziehungsweise das zweite Verbindungsmittel einteilig mit dem dritten Verbindungsmittel ausgeführt sein, und so die Spülflüssigkeit von der Tibiakomponente in die Femur-Komponente eingespeist werden. Bevorzugt werden die Spülflüssigkeitskreisläufe der Tibiakomponente und der Femur-Komponente aus hygienischen Gründen jedoch getrennt voneinander gehalten, damit keine gegenseitige Infizierung auftreten kann.

Bei derartigen Kniespacersystemen kann vorgesehen sein, dass die zumindest eine Spülflüssigkeitsaustrittsöffnung des Femur-Prothesenkörpers und die zumindest eine Spülflüssigkeitseintrittsöffnung des Femur-Prothesenkörpers außerhalb der Kondylen der Femurkomponente angeordnet sind.

Hiermit wird sichergestellt, dass die zumindest eine Spülflüssigkeitsaustrittsöffnung und die zumindest eine Spülflüssigkeitseintrittsöffnung des Femur-Prothesenkörpers nicht die Funktion der Kondylen beeinträchtigen. Zudem kann so eine mechanische Belastung und ein damit einhergehender unerwünschter Abrieb der Kanten der zumindest einen Spülflüssigkeitsaustrittsöffnung des Femur-Prothesenkörpers und der zumindest einen Spülflüssigkeitseintrittsöffnung des Femur-Prothesenkörpers im Bereich der Kondylen verhindert werden.

Schließlich kann vorgesehen sein, dass wenigstens eine Spülflüssigkeitsaustrittsöffnung der zumindest einen Spülflüssigkeitsaustrittsöffnung oder wenigstens eine Spülflüssigkeitseintrittsöffnung der zumindest einen Spülflüssigkeitseintrittsöffnung an einer distalen Seite eines sich in distaler Richtung erstreckenden Vorsprungs zwischen zwei Befestigungsflächen der Tibiakomponente angeordnet ist
Hiermit wird erreicht, dass die Spülflüssigkeit auch den Bereich zur Befestigung in der Tibia erreichen kann.

Der Erfindung liegt die überraschende Erkenntnis zugrunde, dass ein temporäres Kniespacersystem mit einer Tibiakomponente zum kontinuierlichen Spülen einer Kavität im Körper eines Patienten verwendet werden kann, indem an der Oberfläche des Tibia-Prothesenkörpers des Kniespacersystems geeignete Öffnungen und im Inneren des Tibia-Prothesenkörpers geeignete Leitungen für die Spülflüssigkeit vorhanden sind und indem zwei von außen zugängliche Verbindungsmittel angeschlossen sind oder anschließbar sind, durch die die medizinische Spülflüssigkeit von außen in den Tibia-Prothesenkörper eingespeist und die verbrauchte Spülflüssigkeit wieder aus dem Tibia-Prothesenkörper abgeführt werden kann. Bei dem Kniespacersystem können dabei sowohl eine distale als auch eine proximale Seite einer Tibiakomponente durch getrennte Kreisläufe der Spülflüssigkeit gespült werden, so dass das Plateau, das zur Auflage an der Tibia verwendet wird, nicht von der Spülflüssigkeit umflossen werden muss.

Das erfindungsgemäße Kniespacersystem kann vorteilhaft im Rahmen von zweizeitigen septischen Revisionen verwendet werden, bei denen eine Infektion mit zwei oder mehreren mikrobiellen Keimen und insbesondere mit problematischen Keimen vorliegt. Besonders vorteilhaft ist es, dass die Spacerkomponenten und das umgebende Weich- und zumindest teilweise auch das umgebende Knochengewebe mit antibiotisch wirksamen Lösungen, wie Antibiotika und auch Antiseptika oder in speziellen Fällen mit Antimykotika, gespült werden können, wobei die Art und die Anzahl der Wirkstoffe und vor allem die Konzentration der antimikrobiellen Wirkstoffe in der Spülflüssigkeit exakt eingestellt werden können. Durch Absaugen der Spülflüssigkeit kann auch die Verweildauer der antimikrobiell ausgerüsteten Spülflüssigkeit im Patienten exakt eingestellt werden. Dadurch ist es erstmals möglich, über mehrere Tage eine Umspülung der Oberflächen des Kniespacersystems mit exakt zuvor eingestellte Konzentrationen an antimikrobiellen Wirkstoffe in der Spülflüssigkeit zu gewährleisten. Dadurch wird der Schutz vor einer mikrobiellen Wiederbesiedlung der Oberflächen des Kniespacersystems deutlich verringert im Vergleich zu den bisherigen aus Knochenzement gefertigten Spacern. Nach antibiotischer Spülung ist es möglich, die Oberflächen des Kniespacersystems und des umgebenden Gewebes mit einer wirkstofffreien Spülflüssigkeiten zu spülen. Dadurch werden Reste der antimikrobiellen Wirkstoffe entfernt. Eine Resistenzbildung infolge von persistierenden Wirkstoffrückständen ist daher extrem unwahrscheinlich.

Weiterhin ist es vorteilhaft, dass die Spülflüssigkeiten auch solche antimikrobiellen Wirkstoffe enthalten können, die normalerweise nicht in die aus Knochenzement gefertigten Spacern integriert werden können, weil diese die radikalische Polymerisation stören beziehungsweise verhindern würden. Exemplarisch dafür seien die Wirkstoffe Rifampicin und Metronidazol genannt.

Wenn die klinischen Parameter erkennen lassen, dass die Infektion beziehungsweise die Entzündung abklingt, dann können die Verbindungsmittel von der Tibiakomponente entfernt werden. Die Verbindungsmittel sind hierzu vorteilhaft mit einem Außengewinde oder über einen Bajonettverschluss oder über einen Steckverschluss mit der zumindest einen Spülflüssigkeits-Einlassöffnung und der zumindest einen Spülflüssigkeits-Auslassöffnung verbunden. Bevorzugt werden die zumindest eine Spülflüssigkeits-Einlassöffnung und die zumindest eine Spülflüssigkeits-Auslassöffnung dicht nebeneinander oder übereinander neben der Führung der Patella beziehungsweise der Patellarsehne seitlich in der Tibiakomponente angeordnet.

Bevorzugt kann vorgesehen sein, dass die zumindest eine Spülflüssigkeits-Einlassöffnung und die zumindest eine Spülflüssigkeits-Auslassöffnung nach Entfernung der Verbindungsmittel bündig mit der Oberfläche des Tibia-Prothesenkörpers abschließen, um eine Irritation des umgebenden Weichgewebes zu verhindern.

Ein beispielhafter Kniespacer kann zusammengesetzt sein aus
A) einer Tibiakomponente mit zwei Laufflächen auf der proximalen Seite,
B) einer Spülflüssigkeits-Einlassöffnung, die an der Oberfläche der Tibiakomponente angeordnet ist,
C) einem röhrenförmigen, flüssigkeitsdurchlässigen ersten Verbindungsmittel zur Zufuhr der Spülflüssigkeit, das lösbar mit der Spülflüssigkeits-Einlassöffnung verbunden ist,
D) mindestens einer Spülflüssigkeitsaustrittsöffnung an der Oberfläche der Tibiakomponente, die flüssigkeitsdurchlässig über eine erste Leitung im Inneren der Tibiakomponente mit der Spülflüssigkeits-Einlassöffnung verbunden ist,
E) mindestens einer Spülflüssigkeitseintrittsöffnung an der Oberfläche der Tibiakomponente,
F) mindestens einer Spülflüssigkeits-Auslassöffnung an der Oberfläche der Tibiakomponente,
G) einer flüssigkeitsdurchlässigen, röhrenförmigen zweiten Leitung im Inneren der Tibiakomponente, das die Spülflüssigkeitseintrittsöffnung mit der Spülflüssigkeits-Auslassöffnung flüssigkeitsdurchlässig verbindet,
H) einem röhrenförmigen, flüssigkeitsdurchlässigen zweiten Verbindungsmittel zum Absaugen der Spülflüssigkeit, das lösbar mit der Spülflüssigkeits-Auslassöffnung verbunden ist,
I) mindestens eine von einem Steg umschlossene Befestigungsfläche zur Aufnahme von Knochenzement auf der Oberfläche der distalen Seite der Tibiakomponente, und
J) einer Femurkomponente, die zwei Kondylen besitzt, die mit den Laufflächen der Tibiakomponente korrespondieren.

Vorzugsweise ist der Querschnitt der Spülflüssigkeitseintrittsöffnung größer oder gleich dem Querschnitt der Spülflüssigkeits-Einlassöffnung.

Es kann erfindungsgemäß vorgesehen sein, dass mindestens eine Spülflüssigkeitsaustrittsöffnung an der proximalen Seite und mindestens eine Spülflüssigkeitsaustrittsöffnung an der distalen Seite der Tibiakomponente angeordnet sind. Durch die an der proximalen Seite angeordnete Spülflüssigkeitsaustrittsöffnung können die Laufflächen der Tibiakomponente, die lateralen Teile der Tibiakomponente sowie die für die Spülflüssigkeit zugänglichen Teile der Femurkomponente gespült werden. Durch die an der distalen Seite angeordnete Spülflüssigkeitsaustrittsöffnung kann auch das Knochengewebe zumindest teilweise an der distalen Seite der Tibiakomponente gespült werden. Es ist dadurch auch möglich, den proximalen Markraum der Tibia zu spülen, wenn die an der distalen Seite der Tibiakomponente angeordnete Spülflüssigkeitsaustrittsöffnung annähernd zentral angeordnet ist.

Es ist ebenfalls vorteilhaft, wenn mindestens eine Spülflüssigkeitseintrittsöffnung an der proximalen Seite und mindestens eine Spülflüssigkeitseintrittsöffnung an der distalen Seite der Tibiakomponente angeordnet sind. Dadurch ist eine ungehinderte Absaugung der Spülflüssigkeit in beiden Bereichen möglich.

Der Abstand zwischen der Spülflüssigkeitsaustrittsöffnung und der Spülflüssigkeitseintrittsöffnung beträgt mindestens 0,5 cm, bevorzugt mindestens 2,0 cm und ganz besonders bevorzugt mindestens 3,0 cm. Dadurch ist gewährleistet, dass die Spülflüssigkeit ausreichend Debris und Wundsekret aufnehmen kann, bevor sie durch die Spülflüssigkeitseintrittsöffnung über die Leitung wieder abgesaugt wird.

Vorteilhaft kann auch vorgesehen sein, dass die mindestens eine Spülflüssigkeitsaustrittsöffnung und die mindestens eine Spülflüssigkeitseintrittsöffnung auf der proximalen Seite der Tibiakomponente neben den Laufflächen angeordnet sind. Dadurch wird das Gleitverhalten der Tibiakomponente gegen die Femurkomponente nicht beeinträchtigt. Öffnungen in den Laufflächen können bei Gleitpaarungen die Bildung von Abriebpartikel begünstigen und sind deshalb nachteilig oder müssen aufwendig geeignet ausgeführt werden.

Erfindungsgemäß kann auch vorgesehen sein, dass mindestens eine Spülflüssigkeitsaustrittsöffnung und mindestens eine Spülflüssigkeitseintrittsöffnung auf der proximalen und auf der distalen Seite der Tibiakomponente angeordnet sind, wobei die Summe der Querschnitte der Spülflüssigkeitseintrittsöffnungen größer oder gleich dem Querschnitt der Spülflüssigkeitsaustrittsöffnungen ist. Dadurch ist gewährleistet, dass es keine Stauungseffekte bei der Absaugung der Spülflüssigkeit gibt.

Es kann erfindungsgemäß auch vorgesehen sein, dass zwischen der Spülflüssigkeits-Einlassöffnung und dem ersten Verbindungsmittel ein Rückschlagventil angeordnet ist, das ein Zurückfließen der Spülflüssigkeit in das erste Verbindungsmittel verhindert, wobei als Rückschlagventil ein Kugelventil mit Feder, ein Lippenventil, Bunsenventil und ein Plattenventil bevorzugt sind.

Erfindungsgemäß kann vorgesehen sein, dass zwischen der Spülflüssigkeits-Auslassöffnung und dem zweiten Verbindungsmittel ein Rückschlagventil angeordnet ist, das ein Zurückfließen der Spülflüssigkeit in das zweite Verbindungsmittel verhindert, wobei als Rückschlagventil ein Kugelventil mit Feder, ein Lippenventil, Bunsenventil und ein Plattenventil bevorzugt sind.

Weiterhin kann erfindungsgemäß vorgesehen sein, dass zwischen der mindestens einen Spülflüssigkeitsaustrittsöffnung und der angeschlossenen Leitung in dem Tibia-Prothesenkörper ein Ventil angeordnet ist, das nur durch Anlegen eines Unterdrucks an die Leitung zu öffnen ist, damit ein Zurückfließen der verbrauchten Spülflüssigkeit in die Leitung verhindert wird, wobei als Ventil ein Kugelventil mit Feder, ein Lippenventil, Bunsenventil und ein Plattenventil bevorzugt sind.

Ebenso kann vorgesehen sein, dass zwischen der mindestens einen Spülflüssigkeitseintrittsöffnung und der angeschlossenen Leitung in dem Tibia-Prothesenkörper ein Ventil angeordnet ist, das nur durch Anlegen eines Unterdrucks an die Leitung zu öffnen ist, damit ein Zurückfließen der verbrauchten Spülflüssigkeit in die Leitung verhindert wird, wobei als Ventil ein Kugelventil mit Feder, ein Lippenventil, Bunsenventil und ein Plattenventil bevorzugt sind.

Vorteilhaft kann auch vorgesehen sein, dass auf der Oberfläche der distalen Seite der Femurkomponente zwei oder mehrere von Stegen eingegrenzte Flächen zur Aufnahme von Knochenzement angeordnet sind. Dadurch kann die Spülflüssigkeit auf das Knochengewebe am distalen Femur einwirken, dass nicht vom Knochenzement abgedeckt ist.

Das erfindungsgemäße Kniespacersystem kann aus Kunststoff, Metall, Keramik, Glaskeramik und deren Kombinationen gefertigt sein. Vorteilhaft kann das Kniespacersystem auch aus ausgehärtetem Knochenzement bestehen. Der Knochenzement kann zusätzlich antimikrobielle Wirkstoffe, wie Antiseptika, Antibiotika, Antimykotika enthalten.

Im Folgenden werden weitere Ausführungsbeispiele der Erfindung anhand von fünfzehn schematisch dargestellten Figuren erläutert, ohne jedoch dabei die Erfindung zu beschränken.

Dabei zeigt:
Figur 1: eine schematische perspektivische Ansicht auf die proximale Seite einer Tibiakomponente als erstes erfindungsgemäßes Kniespacersystem;
Figur 2: eine schematische perspektivische Ansicht auf die distale Seite der Tibiakomponente nach Figur 1;
Figur 3: eine schematische Seitenansicht auf eine laterale Seite der Tibiakomponente nach den Figuren 1 und 2;
Figur 4: eine seitliche Querschnittansicht der Tibiakomponente entlang einer Sagittalebene der Tibiakomponente nach den Figuren 1 bis 3;
Figur 5: eine zweite Querschnittansicht der Tibiakomponente nach den Figuren 1 bis 4;
Figur 6: eine dritte Querschnittansicht der Tibiakomponente nach den Figuren 1 bis 5;
Figur 7: eine schematische perspektivische Ansicht auf die proximale Seite einer beispielhaften Femurkomponente für ein erfindungsgemäßes Kniespacersystem;
Figur 8: eine schematische perspektivische Ansicht auf die proximale Seite einer eines beispielhaften zweiten erfindungsgemäßen Kniespacersystems mit einer Tibiakomponente (unten) und einer Femurkomponente (oben);
Figur 9: zwei schematische perspektivische Ansichten auf die proximale Seite der Tibiakomponente des zweiten erfindungsgemäßen Kniespacersystems nach Figur 8 mit unterschiedlich belegten Anschlüssen;
Figur 10: zwei schematische perspektivische Ansichten auf die distale Seite der Tibiakomponente nach Figur 9;
Figur 11: zwei schematische Aufsichten auf die proximale Seite der Tibiakomponente nach den Figuren 9 und 10;
Figur 12: zwei Querschnittansichten entlang einer Transversalebene der Tibiakomponente nach den Figuren 9 bis 11;
Figur 13: zwei weitere Querschnittansichten entlang einer zu Figur 12 parallelen Transversalebene der Tibiakomponente nach den Figuren 9 bis 12;
Figur 14: eine schematische Seitenansicht auf eine laterale Seite der Tibiakomponente nach den Figuren 8 bis 13; und
Figur 15: eine seitliche Querschnittansicht entlang einer Sagittalebene der Tibiakomponente nach den Figuren 8 bis 14.

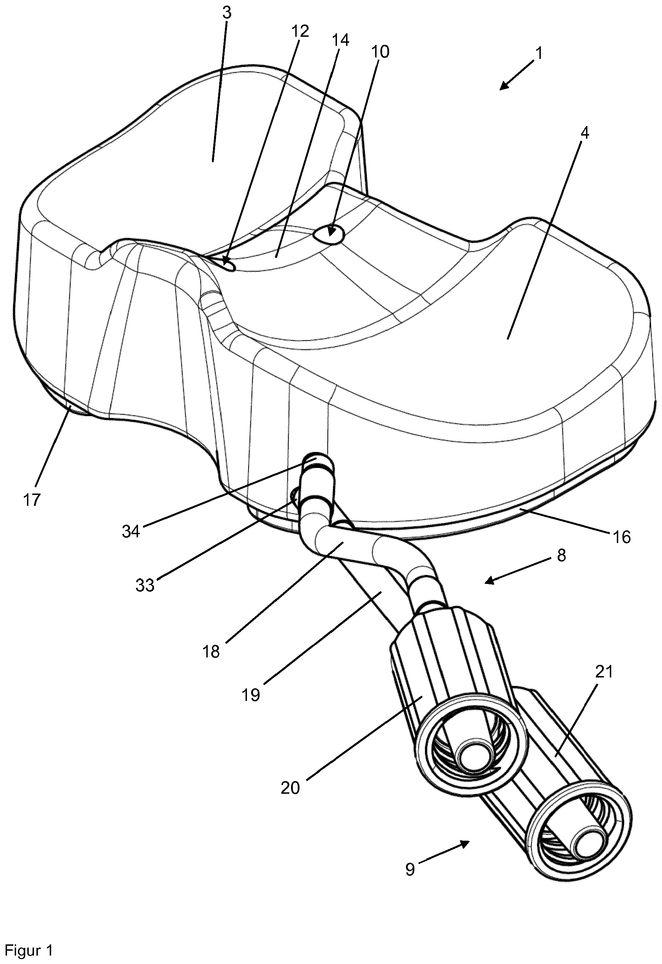

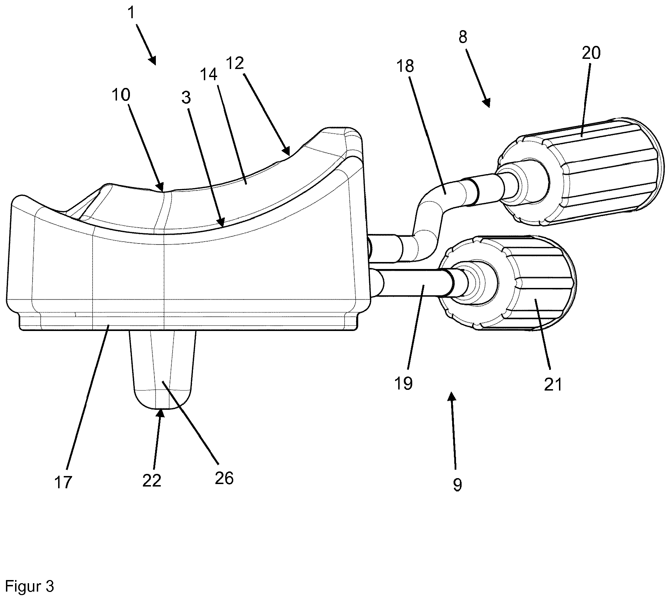

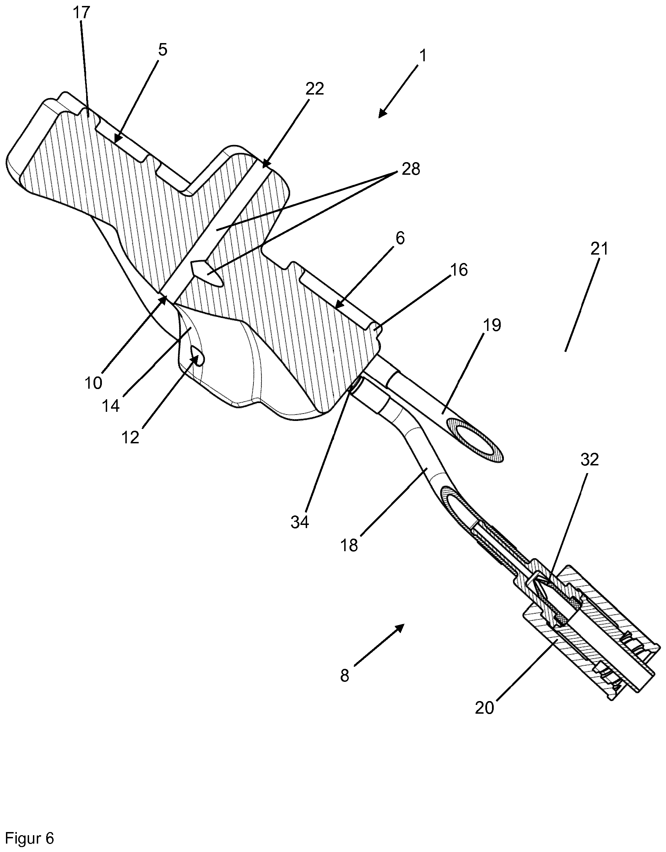

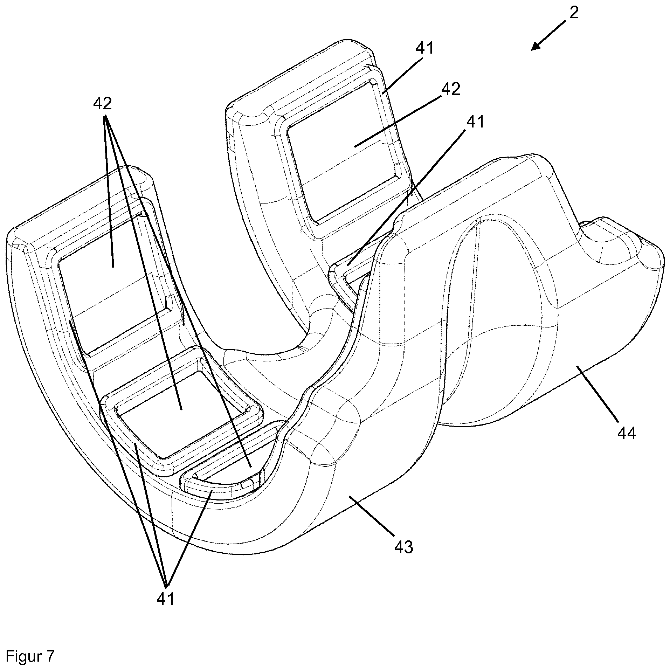

In den Figuren 1 bis 6 sind Abbildungen einer Tibiakomponente 1 als erstes erfindungsgemäßes Kniespacersystems mit Spülvorrichtung gezeigt. Figur 7 zeigt dazu eine Femurkomponente 2, die zusammen mit der Tibiakomponente 1 nach den Figuren 1 bis 6 ein erfindungsgemäßes Kniespacersystem für ein vollständiges Kniegelenk bilden kann.

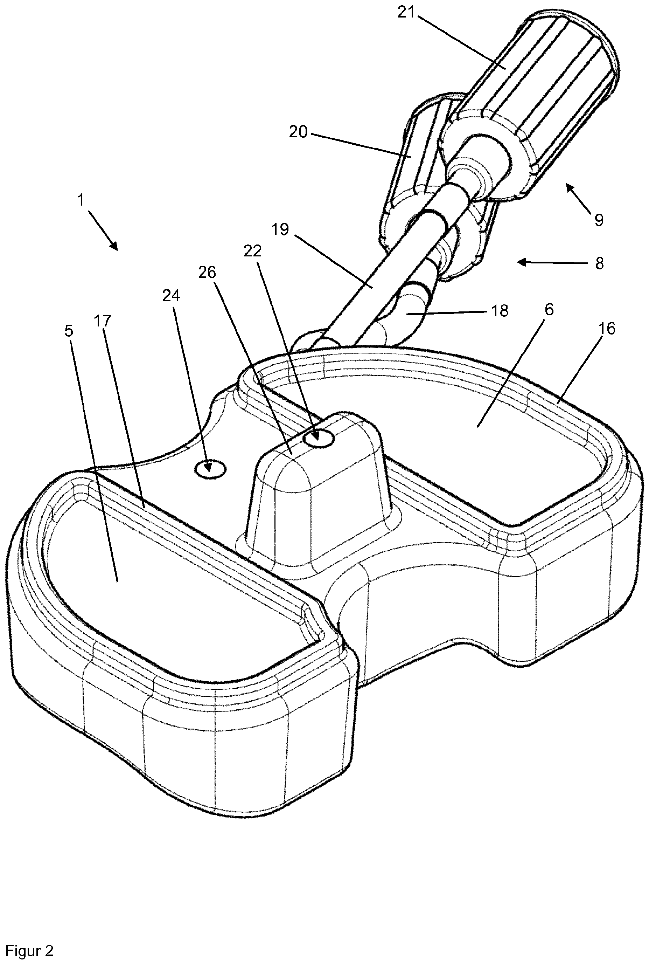

Die Tibiakomponente 1 umfasst auf ihrer proximalen Seite zwei Laufflächen 3, 4 (siehe insbesondere Figur 1). Die Laufflächen 3, 4 bilden eine Abrollfläche des Kniegelenks beziehungsweise des Kniespacersystems. Auf der gegenüberliegenden distalen Seite der Tibiakomponente 1 sind zwei Befestigungsflächen 5, 6 angeordnet (siehe insbesondere Figur 2), die zur Verbindung der Tibiakomponente 1 mit einer Tibia (nicht gezeigt) mit Hilfe von Knochenzementteig vorgesehen sind. Die Laufflächen 3, 4 und die Befestigungsflächen 5, 6 sind an einem Tibia-Prothesenkörper angeordnet, dessen äußere Form im Wesentlichen der äußeren Form einer Tibiakomponente eines herkömmlichen Kniespacers entspricht.

An einer seitlichen Oberfläche des Tibia-Prothesenkörpers sind im Unterschied zu Tibiakomponenten bekannter Kniespacersysteme ein erstes röhrenförmiges Verbindungsmittel 8 zum Zuführen einer Spülflüssigkeit an einer Spülflüssigkeits-Einlassöffnung befestigt und ein zweites röhrenförmiges Verbindungsmittel 9 zum Abführen einer Spülflüssigkeit an einer Spülflüssigkeits-Auslassöffnung angeschlossen. Die Spülflüssigkeits-Einlassöffnung und die Spülflüssigkeits-Auslassöffnung sind im Tibia-Prothesenkörper angeordnet und führen ins Innere des Tibia-Prothesenkörpers. Das erste röhrenförmige Verbindungsmittel 8 und das zweite röhrenförmige Verbindungsmittel 9 sind flüssigkeitsdurchlässig, so dass eine medizinische Spülflüssigkeit durch das erste röhrenförmige Verbindungsmittel 8 in den Tibia-Prothesenkörper hineingeleitet werden kann und eine Flüssigkeit durch das zweite röhrenförmige Verbindungsmittel 9 aus dem Tibia-Prothesenkörper abgeführt werden kann. Das erste Verbindungsmittel 8 und das zweite Verbindungsmittel 9 sind lösbar mit der Spülflüssigkeits-Einlassöffnung und der Spülflüssigkeits-Auslassöffnung verbunden.

An der proximalen Oberfläche der Tibiakomponente 1 sind eine Spülflüssigkeitsaustrittsöffnung 10 und eine Spülflüssigkeitseintrittsöffnung 12 auf einem Sattel 14 zwischen den Laufflächen 3, 4 angeordnet. Die Befestigungsflächen 5, 6 sind jeweils begrenzt durch je einen umlaufenden Steg 16, 17. Die Stege 16, 17 erheben sich aus der distalen Oberfläche der Tibiakomponente 1 und sind als Teil des Tibia-Prothesenkörpers zu verstehen.

Das erste Verbindungsmittel 8 hat einen kurzen, flexiblen Schlauch 18 und einen Luer-Lock-Adapter 20. Ebenso hat das zweite Verbindungsmittel 9 einen kurzen, flexiblen Schlauch 19 und einen Luer-Lock-Adapter 21. Dadurch kann die Tibiakomponente 1 mit dem ersten Verbindungsmittel 8 über den Luer-Lock-Adapter 20 bequem an eine Quelle für eine medizinische Spülflüssigkeit mit einer Pumpe (nicht gezeigt) angeschlossen werden und das zweite Verbindungsmittel 9 über den Luer-Lock-Adapter 21 an einen Sammelbehälter und gegebenenfalls ebenfalls eine Pumpe (nicht gezeigt).

Durch die vorstehenden Stege 16, 17 um die Befestigungsflächen 5, 6 wird verhindert oder zumindest erschwert, dass beim Befestigen der Tibia-Komponente 1 an der Tibia Knochenzementteig außerhalb der Befestigungsflächen 5, 6 vordringt und dadurch die proximale Spülflüssigkeitsaustrittsöffnung 10, die proximale Spülflüssigkeitseintrittsöffnung 12, die distale Spülflüssigkeitsaustrittsöffnung 22, die distale Spülflüssigkeitseintrittsöffnung 24 oder die Spülflüssigkeits-Einlassöffnung und die Spülflüssigkeits-Auslassöffnung verschließt oder beeinträchtigt beziehungsweise ungewollt das erste Verbindungsmittel 8 oder das zweite Verbindungsmittel 9 am Tibia-Prothesenkörper festzementiert.

An der distalen Oberfläche der Tibiakomponente 1 sind eine Spülflüssigkeitsaustrittsöffnung 22 und eine Spülflüssigkeitseintrittsöffnung 24 zwischen den Befestigungsflächen 5, 6 angeordnet. Die distale Spülflüssigkeitsaustrittsöffnung 22 ist auf einem distalen Vorsprung 26 angeordnet. Der distale Vorsprung 26 ist zur Verankerung der Tibiakomponente 1 beziehungsweise des Tibia-Prothesenkörpers in der Tibia vorgesehen.

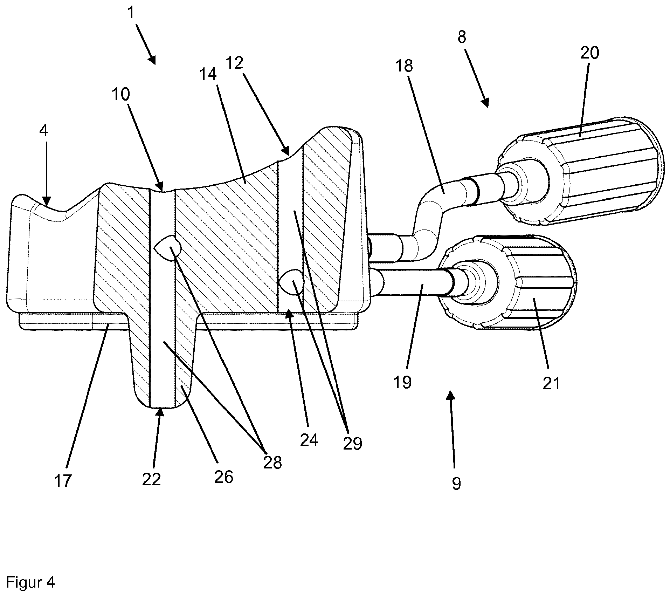

In den Querschnittansichten nach den Figuren 4, 5 und 6 ist weitgehend zu erkennen, wie die die Spülflüssigkeits-Auslassöffnung mit der proximalen Spülflüssigkeitseintrittsöffnung 12 und der distalen Spülflüssigkeitseintrittsöffnung 24 und die Spülflüssigkeits-Einlassöffnung mit der proximalen Spülflüssigkeitsaustrittsöffnung 10 und der distalen Spülflüssigkeitsaustrittsöffnung 22 im Inneren des Tibia-Prothesenkörpers verbunden sind. Der Tibia-Prothesenkörper ist im Wesentlichen aus einem Kunststoff gefertigt. Bevorzugt aus einem Knochenzement, wie einem PMMA-Kunststoff, der mit einem Antibiotikum oder mit mehreren Antibiotika und/oder mit zumindest einem Antimykotikum beladen sein kann.

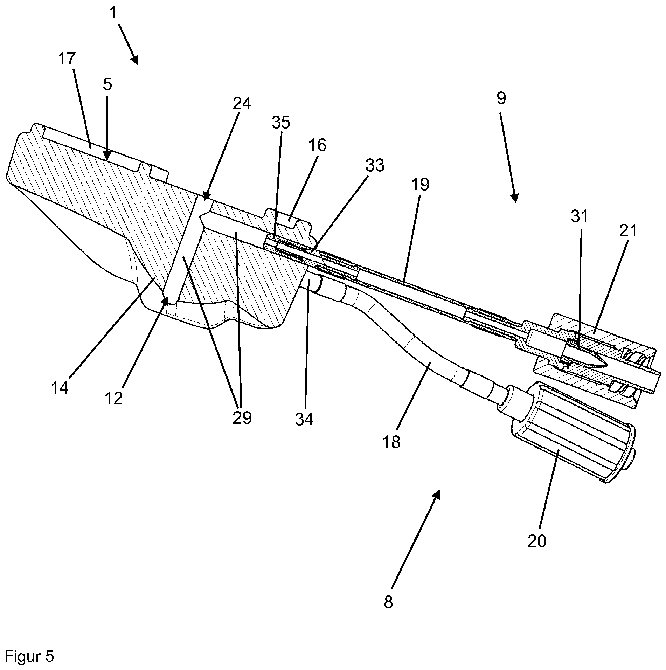

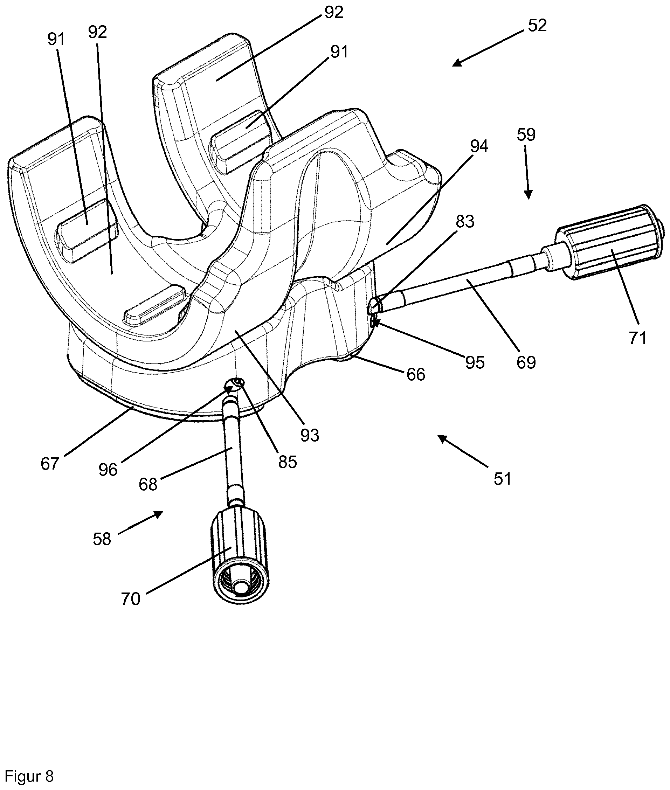

Im Inneren des Tibia-Prothesenkörpers ist die Spülflüssigkeits-Einlassöffnung mit der proximalen Spülflüssigkeitsaustrittsöffnung 10 und der distalen Spülflüssigkeitsaustrittsöffnung 22 über eine erste Leitung 28 verbunden. Die erste Leitung 28 stellt eine flüssigkeitsdurchlässige Verbindung zwischen der Spülflüssigkeits-Einlassöffnung und der proximalen Spülflüssigkeitsaustrittsöffnung 10 und der distalen Spülflüssigkeitsaustrittsöffnung 22 her. Hierzu ist im Inneren des Tibia-Prothesenkörpers ein Abzweig in Form eines T-Stücks in der ersten Leitung 28 vorhanden (siehe Figuren 4 und 8). Ebenso ist im Inneren des Prothesenkörpers die Spülflüssigkeits-Auslassöffnung mit der proximalen Spülflüssigkeitseintrittsöffnung 12 und der distalen Spülflüssigkeitseintrittsöffnung 24 über eine zweite Leitung 29 verbunden. Auch die zweite Leitung 29 umfasst zu diesem Zweck einen Abzweig in Form eines T-Stücks (siehe die Figuren 4 und 5). Die erste Leitung 28 und die zweite Leitung 29 sind im Inneren des Tibia-Prothesenkörpers voneinander getrennt.

Im Luer-Lock-Adapter 21 des zweiten Verbindungsmittels 9 zum Abführen der Spülflüssigkeit ist ein Ventilelement 31 vorgesehen, das einen Abfluss von Flüssigkeit aus der zweiten Leitung 29 durch die Spülflüssigkeits-Auslassöffnung aus dem Tibia-Prothesenkörper und durch das zweite Verbindungsmittel 9 erlaubt und das einen Rückfluss aus einer an den Luer-Lock-Adapter 21 angeschlossenen Ableitung in den Schlauch 19 des zweiten Verbindungsmittels 9 in die zweite Leitung 29 hinein verhindert. Ebenso ist im Luer-Lock-Adapter 20 des ersten Verbindungsmittels 8 zum Zuführen einer Spülflüssigkeit ein weiteres Ventilelement 32 vorgesehen, das einen Zufluss von medizinischer Spülflüssigkeit durch das erste Verbindungsmittel 8 und durch die Spülflüssigkeits-Einlassöffnung in die erste Leitung 28 und den Tibia-Prothesenkörper hinein erlaubt und das einen Rückfluss aus dem Schlauch 18 des ersten Verbindungsmittels 8 in eine an den Luer-Lock-Adapter 20 angeschlossenen Quelle für die medizinische hinein verhindert.

Das zweite Verbindungsmittel 9 ist über ein lösbares Verbindungselement 33 mit der Spülflüssigkeits-Auslassöffnung verbunden (siehe Figur 5). In gleicher Weise ist das erste Verbindungsmittel 8 über ein lösbares Verbindungselement 34 mit der Spülflüssigkeits-Einlassöffnung verbunden. Das lösbare Verbindungselement 34 ist von außen nur ansatzweise zu erkennen (siehe Figur 1). Zur Verbindung des ersten Verbindungsmittels 8 und des zweiten Verbindungsmittels 9 mit dem Tibia-Prothesenkörper sind in der ersten Leitung 28 ein erstes Gegenbefestigungselement (in den Figuren nicht zu sehen aber vorhanden) und in der zweiten Leitung 29 in gleicher Weise ein zweites Gegenbefestigungselement 35 angeordnet. Die Verbindungselemente 33, 34 können über ein Außengewinde an einem Innengewinde der beiden Gegenbefestigungselemente 35 befestigt werden beziehungsweise sein. Alternativ kann auch ein Bajonett-Verschluss oder eine Steckverbindung realisiert werden. Das erste Verbindungsmittel 8 und das zweite Verbindungsmittel 9 können so durch abziehen oder abschrauben der lösbaren Verbindungselemente 33, 34 von dem Tibia-Prothesenkörper gelöst werden. Das erste Gegenbefestigungselement und das zweite Gegenbefestigungselement 35 sind vorzugsweise derart ausgeführt, dass sie die Spülflüssigkeits-Auslassöffnung und die Spülflüssigkeits-Einlassöffnung automatisch verschließen, wenn die Verbindungsmittel 33, 34 gelöst werden.

Figur 7 zeigt eine schematische perspektivische Ansicht auf die proximale Seite der beispielhaften Femurkomponente 2, die zusammen mit der Tibiakomponente 1 zur Ausbildung eines erfindungsgemäßen Kniespacersystems zum Ersetzen eines vollständigen Kniegelenks geeignet ist. Die Femurkomponente 2 weist auf ihrer proximalen Seite mehrere von Stegen 41 begrenzte Befestigungsflächen 42 auf. Die Befestigungsflächen 42 dienen der Verbindung der Femurkomponente 2 mit einem Femur, wobei ein Knochenzement die Befestigungsflächen 42 mit dem Femur verbindet. Die Stege 41 ragen in proximaler Richtung aus der proximalen Oberfläche der Femurkomponente 2 hervor und verhindern so ein Vordringen von Knochenzement zwischen die Befestigungsflächen 42. Dadurch bleiben die Zwischenräume zwischen den Befestigungsflächen 42 frei und können von der Spülflüssigkeit durchspült werden, so dass auch der Zwischenraum zwischen der Femurkomponente 2 und dem Femur mit der Spülflüssigkeit erreichbar ist.

An der distalen Seite hat die Femurkomponente 2 zwei nebeneinander angeordnete Kondylen 43, 44. Die Kondylen 43, 44 können auf den Laufflächen der Tibiakomponente 1 abrollen, so dass die Femurkomponente 2 und die Tibiakomponente 1 zusammen ein Kniespacersystem bilden. Alternativ kann aber auch eine andere Femurkomponente zusammen mit der Tibiakomponente 1 nach den Figuren 1 bis 6 zur Bildung eines Kniespacersystems verwendet werden, wie beispielsweise die in Figur 8 gezeigte Femurkomponente 52 oder eine andere bereits bekannte Femurkomponente.

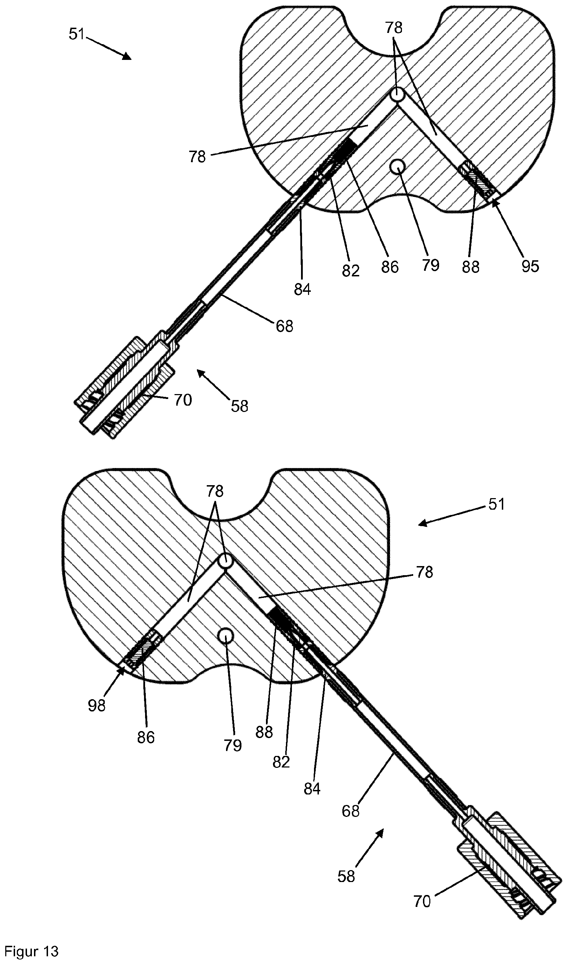

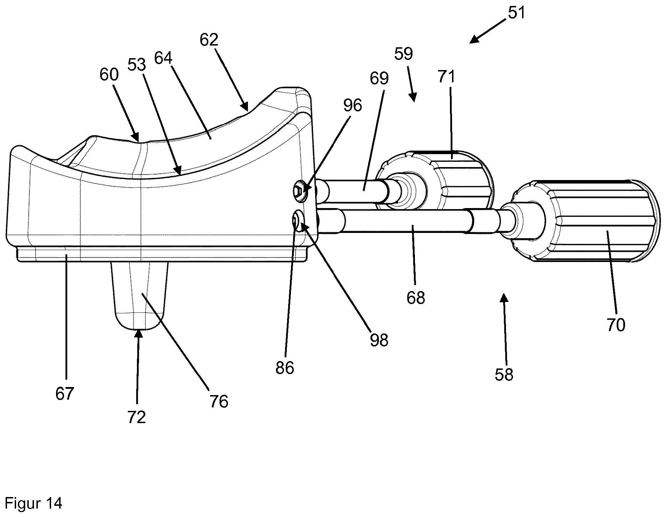

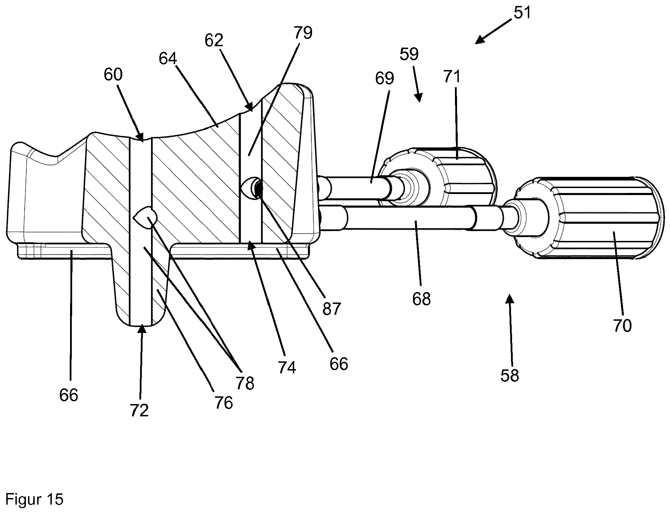

In den Figuren 8 bis 15 sind Abbildungen einer Tibiakomponente 51 eines zweiten alternativen erfindungsgemäßen Kniespacersystems mit Spülvorrichtung gezeigt. Dabei zeigt Figur 8 zusätzlich eine Femurkomponente 52, die zusammen mit der Tibiakomponente 51 das Kniespacersystem bildet. In Figur 8 sind die Tibiakomponente 51 und die Femurkomponente 52 bestimmungsgemäß aneinander liegend dargestellt, so wie das Kniespacersystem auch bei einem beim Patienten eingesetzten Zustand liegen würde.

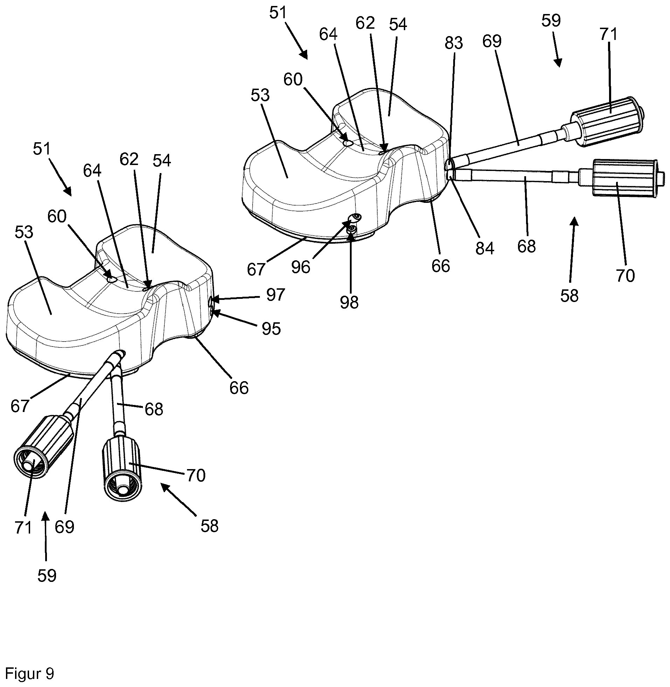

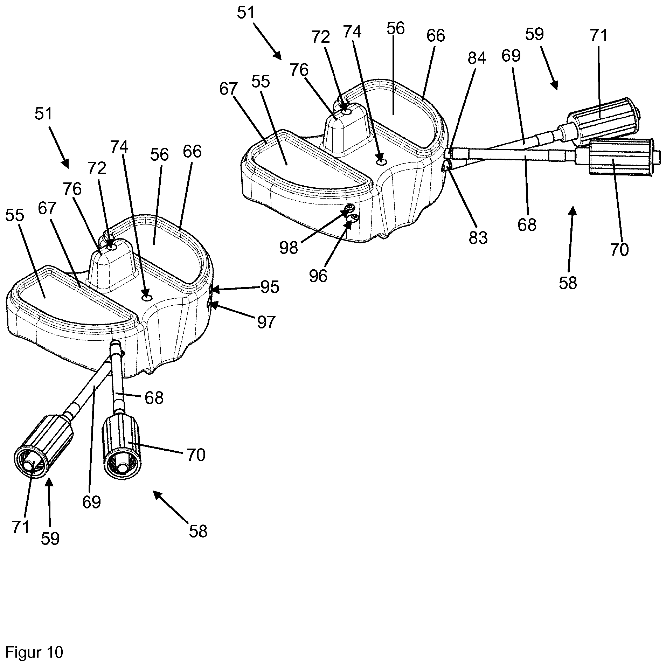

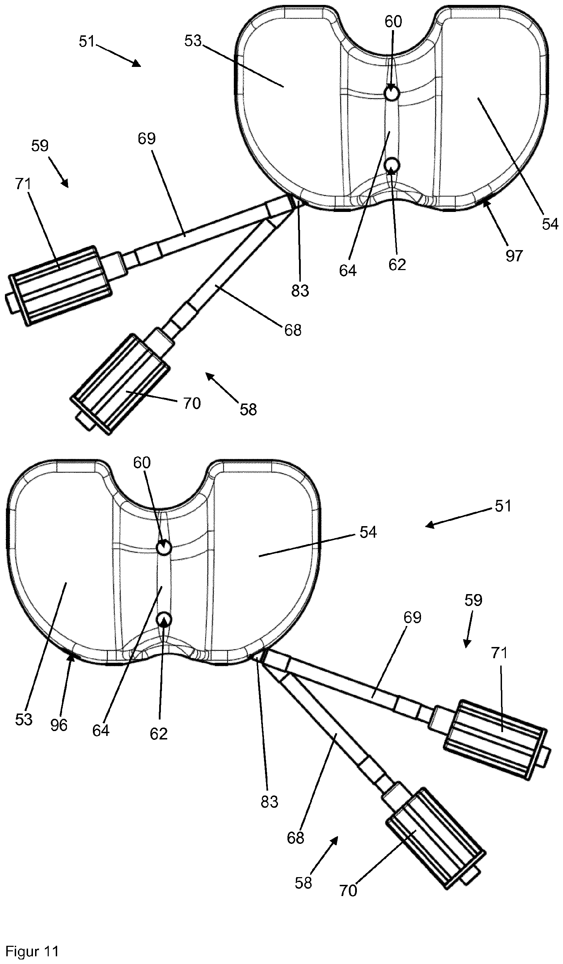

Die Tibiakomponente 51 umfasst auf ihrer proximalen Seite zwei Laufflächen 53, 54 (siehe Figuren 9 und 11), auf denen die Femurkomponente 52 in Figur 8 aufliegt. Die Laufflächen 53, 54 bilden eine Abrollfläche des Kniegelenks beziehungsweise des Kniespacersystems. Auf der gegenüberliegenden distalen Seite der Tibiakomponente 51 sind zwei Befestigungsflächen 55, 56 angeordnet (siehe insbesondere Figur 10), die zur Verbindung der Tibiakomponente 51 mit der Tibia mit Hilfe von Knochenzementteig (nicht gezeigt) vorgesehen sind. Die Laufflächen 53, 54 und die Befestigungsflächen 55, 56 sind an einem Tibia-Prothesenkörper angeordnet, dessen äußere Form im Wesentlichen der äußeren Form einer Tibiakomponente eines herkömmlichen Kniespacers entspricht.

An einer seitlichen Oberfläche des Tibia-Prothesenkörpers sind im Unterschied zu Tibiakomponenten bekannter Kniespacersysteme ein erstes röhrenförmiges Verbindungsmittel 58 zum Zuführen einer Spülflüssigkeit an einer von zwei Spülflüssigkeits-Einlassöffnungen 95, 98 angeschlossen oder anschließbar und ein zweites röhrenförmiges Verbindungsmittel 59 zum Abführen einer Spülflüssigkeit an einer von zwei Spülflüssigkeits-Auslassöffnungen 96, 97 angeschlossen. Die Spülflüssigkeits-Einlassöffnungen 95, 98 und die Spülflüssigkeits-Auslassöffnungen 96, 97 sind im Tibia-Prothesenkörper angeordnet und führen ins Innere des Tibia-Prothesenkörpers. Die paarweise Anordnung jeweils zweier Spülflüssigkeits-Einlassöffnungen 95, 98 und Spülflüssigkeits-Auslassöffnungen 96, 97 ermöglicht ein an die individuelle Situation angepasstes Anschließen des ersten Verbindungsmittels 58 und des zweiten Verbindungsmittels 59. Zudem kann so die Tibiakomponente 51 problemlos sowohl für das rechte als auch für das linke Knie verwendet werden und gleichzeitig die Verbindungsmittel 58, 59 lateral nach außen geführt werden, wo sie weniger stören. Die zweite Ausführungsform unterscheidet sich von der ersten Ausführungsform nach den Figuren 1 bis 6 vor allem durch die größere Anzahl von Öffnungen 95, 96, 97, 98 und die dadurch erreichte Variabilität. Das erste röhrenförmige Verbindungsmittel 58 und das zweite röhrenförmige Verbindungsmittel 59 sind flüssigkeitsdurchlässig, so dass eine medizinische Spülflüssigkeit durch das erste röhrenförmige Verbindungsmittel 58 in den Tibia-Prothesenkörper hineingeleitet werden kann und eine Flüssigkeit durch das zweite röhrenförmige Verbindungsmittel 59 aus dem Tibia-Prothesenkörper abgeführt werden kann. Das erste Verbindungsmittel 58 und das zweite Verbindungsmittel 59 sind lösbar und variabel mit einer der Spülflüssigkeits-Einlassöffnungen 95, 98 und mit einer der Spülflüssigkeits-Auslassöffnungen 96, 97 verbunden.

An der proximalen Oberfläche der Tibiakomponente 51 sind eine Spülflüssigkeitsaustrittsöffnung 60 und eine Spülflüssigkeitseintrittsöffnung 62 auf einem Sattel 64 zwischen den Laufflächen 53, 54 angeordnet. Die Befestigungsflächen 55, 56 sind jeweils begrenzt durch je einen umlaufenden Steg 66, 67. Die Stege 66, 67 erheben sich aus der distalen Oberfläche der Tibiakomponente 51 und sind als Teil des Tibia-Prothesenkörpers zu verstehen.

Das erste Verbindungsmittel 58 hat einen kurzen, flexiblen Schlauch 68 und einen Luer-Lock-Adapter 70. Ebenso hat das zweite Verbindungsmittel 59 einen kurzen, flexiblen Schlauch 69 und einen Luer-Lock-Adapter 71. Dadurch kann die Tibiakomponente 51 mit dem ersten Verbindungsmittel 58 über den Luer-Lock-Adapter 70 problemlos an eine Quelle für eine medizinische Spülflüssigkeit mit einer Pumpe (nicht gezeigt) angeschlossen werden und das zweite Verbindungsmittel 59 über den Luer-Lock-Adapter 71 an einen Sammelbehälter und gegebenenfalls ebenfalls eine Pumpe (nicht gezeigt).

An der distalen Oberfläche der Tibiakomponente 51 sind eine Spülflüssigkeitsaustrittsöffnung 72 und eine Spülflüssigkeitseintrittsöffnung 74 zwischen den Befestigungsflächen 55, 56 angeordnet. Die distale Spülflüssigkeitsaustrittsöffnung 72 ist auf einem distalen Vorsprung 76 angeordnet. Der distale Vorsprung 76 ist zur Verankerung der Tibiakomponente 51 beziehungsweise des Tibia-Prothesenkörpers in der Tibia vorgesehen.

Durch die vorstehenden Stege 66, 67 um die Befestigungsflächen 55, 56 wird verhindert oder zumindest erschwert, dass beim Befestigen der Tibia-Komponente 51 an der Tibia Knochenzementteig außerhalb der Befestigungsflächen 55, 56 vordringt und dadurch die proximale Spülflüssigkeitsaustrittsöffnung 60, die proximale Spülflüssigkeitseintrittsöffnung 62, die distale Spülflüssigkeitsaustrittsöffnung 72, die distale Spülflüssigkeitseintrittsöffnung 74 oder die Spülflüssigkeits-Einlassöffnung und die Spülflüssigkeits-Auslassöffnung verschließt oder beeinträchtigt beziehungsweise ungewollt das erste Verbindungsmittel 58 oder das zweite Verbindungsmittel 59 am Tibia-Prothesenkörper festzementiert.

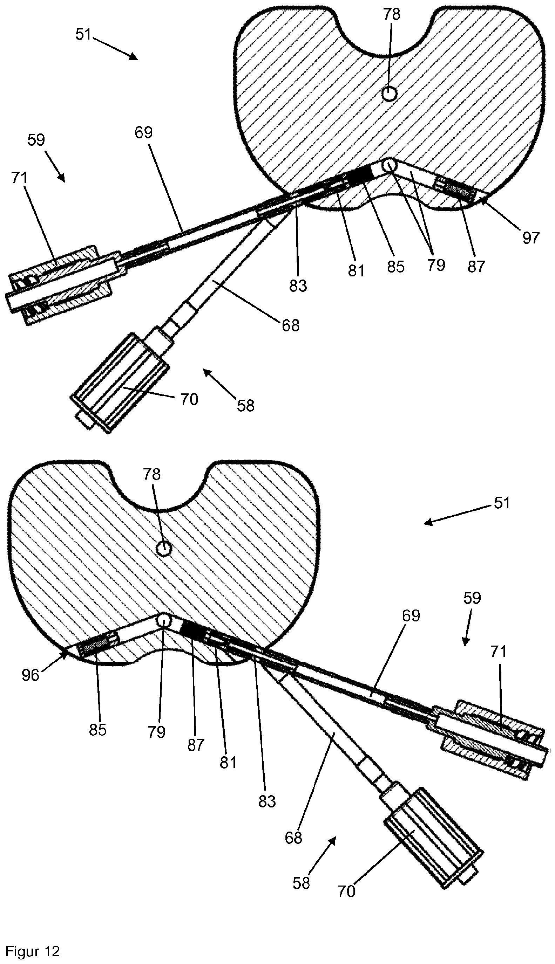

In den Querschnittansichten nach den Figuren 12, 13 und 15 ist weitgehend zu erkennen, wie die die Spülflüssigkeits-Auslassöffnung mit der proximalen Spülflüssigkeitseintrittsöffnung 62 und der distalen Spülflüssigkeitseintrittsöffnung 74 und die Spülflüssigkeits-Einlassöffnung mit der proximalen Spülflüssigkeitsaustrittsöffnung 60 und der distalen Spülflüssigkeitsaustrittsöffnung 72 im Inneren des Tibia-Prothesenkörpers verbunden sind. Der Tibia-Prothesenkörper ist im Wesentlichen aus einem Kunststoff gefertigt. Bevorzugt aus einem Knochenzement, wie einem PMMA-Kunststoff, der mit einem Antibiotikum oder mit mehreren Antibiotika beladen sein kann.

Im Inneren des Tibia-Prothesenkörpers ist die Spülflüssigkeits-Einlassöffnung mit der proximalen Spülflüssigkeitsaustrittsöffnung 60 und der distalen Spülflüssigkeitsaustrittsöffnung 72 über eine erste Leitung 78 verbunden. Die erste Leitung 78 stellt eine flüssigkeitsdurchlässige Verbindung zwischen der Spülflüssigkeits-Einlassöffnung und der proximalen Spülflüssigkeitsaustrittsöffnung 60 und der distalen Spülflüssigkeitsaustrittsöffnung 72 her. Hierzu ist im Inneren des Tibia-Prothesenkörpers ein Abzweig in Form eines T-Stücks in der ersten Leitung 78 vorhanden (siehe Figuren 12, 13 und 15). Ebenso ist im Inneren des Prothesenkörpers die Spülflüssigkeits-Auslassöffnung mit der proximalen Spülflüssigkeitseintrittsöffnung 62 und der distalen Spülflüssigkeitseintrittsöffnung 74 über eine zweite Leitung 79 verbunden. Auch die zweite Leitung 79 umfasst zu diesem Zweck einen Abzweig in Form eines T-Stücks. Die erste Leitung 78 und die zweite Leitung 79 sind im Inneren des Tibia-Prothesenkörpers voneinander getrennt.

An einem dem Luer-Lock-Adapter 20 gegenüberliegenden Ende des ersten Verbindungsmittels 58 zum Zuführen einer Spülflüssigkeit ist ein erstes Ventilelement 82 an einem Verbindungselement 84 angeordnet, das einen Zufluss von medizinischer Spülflüssigkeit aus dem ersten Verbindungsmittel 58 in die erste Leitung 78 und in den Tibia-Prothesenkörper hinein erlaubt und das einen Rückfluss aus der ersten Leitung 78 in das Verbindungsmittel 58 hinein verhindert. Ebenso ist an einem dem Luer-Lock-Adapter 71 gegenüberliegenden Ende des zweiten Verbindungsmittels 59 zum Abführen einer Spülflüssigkeit ein zweites Ventilelement 81 in einem zweiten Verbindungselement 83 vorgesehen, das einen Abfluss von Flüssigkeit aus der zweiten Leitung 79 in das zweite Verbindungsmittel 59 erlaubt und das einen Rückfluss aus dem Schlauch 69 in die zweite Leitung 79 hinein verhindert. Das erste Ausführungsbeispiel nach den Figuren 1 bis 6 unterscheidet sich also von dem zweiten Ausführungsbeispiel nach den Figuren 8 bis 15 auch durch die Anordnung der Ventilelemente 81, 82 in den Verbindungsmitteln 58, 59.

Das zweite Verbindungsmittel 59 ist über das lösbare Verbindungselement 83 mit jeder der Spülflüssigkeits-Auslassöffnungen 96, 97 verbunden oder verbindbar und, wenn dies gewünscht ist, auch mit jeder der Spülflüssigkeits-Einlassöffnungen 95, 98 verbindbar. In gleicher Weise ist das erste Verbindungsmittel 58 über das lösbare Verbindungselement 84 mit jeder der Spülflüssigkeits-Einlassöffnungen 95, 98 verbunden oder verbindbar und, wenn dies gewünscht ist, auch mit jeder der Spülflüssigkeits-Auslassöffnungen 96, 97 verbindbar. Zur Verbindung des ersten Verbindungsmittels 58 mit dem Tibia-Prothesenkörper sind in der ersten Leitung 78 im Bereich der ersten Spülflüssigkeits-Einlassöffnung 95 ein erstes Gegenbefestigungselement 88 und im Bereich der zweiten Spülflüssigkeits-Einlassöffnung 98 ein zweites Gegenbefestigungselement 86 angeordnet. In gleicher Weise sind zur Verbindung des zweiten Verbindungsmittels 59 mit dem Tibia-Prothesenkörper in der zweiten Leitung 79 im Bereich der ersten Spülflüssigkeits-Auslassöffnung 96 ein drittes Gegenbefestigungselement 85 und im Bereich der zweiten Spülflüssigkeits-Auslassöffnung 97 ein viertes Gegenbefestigungselement 87 angeordnet. Die Verbindungselemente 83, 84 können über ein Außengewinde an einem der Gegenbefestigungselemente 85, 86, 87, 88 befestigt werden beziehungsweise sein. Die Befestigung kann beispielsweise über Gewinde erfolgen oder auch durch einen Bajonett-Verschluss oder eine Steckverbindung realisiert werden. Das erste Verbindungsmittel 58 und das zweite Verbindungsmittel 59 können so durch abziehen oder abschrauben der lösbaren Verbindungselemente 83, 84 von dem Tibia-Prothesenkörper gelöst werden. Die Gegenbefestigungselemente 85, 86, 87, 88 sind derart ausgeführt, dass sie die Spülflüssigkeits-Auslassöffnungen 96, 97 und die Spülflüssigkeits-Einlassöffnungen 95, 98 automatisch verschließen, wenn die Verbindungsmittel 83, 84 gelöst werden.

Die Femurkomponente 52 weist auf ihrer proximalen Seite mehrere von Stegen 91 unterbrochene Befestigungsflächen 92 auf. Die Befestigungsflächen 92 dienen der Verbindung der Femurkomponente 52 mit einem Femur, wobei ein Knochenzement die Befestigungsflächen 92 mit dem Femur verbindet. Die Stege 91 ragen in proximaler Richtung aus der proximalen Oberfläche der Femurkomponente 52 hervor. Alternativ kann auch die Femurkomponente 2 gemäß Figur 7 verwendet werden, um ein Kniespacersystem zu realisieren.

An der distalen Seite hat die Femurkomponente 52 zwei nebeneinander angeordnete Kondylen 93, 94. Die Kondylen 93, 94 können auf den Laufflächen der Tibiakomponente 51 abrollen, so dass die Femurkomponente 52 und die Tibiakomponente 51 zusammen ein Kniespacersystem bilden.

Gemäß einer weiteren, nicht in den Figuren dargestellten Ausführung kann auch die Femurkomponente mit einer Spülvorrichtung ausgerüstet werden. Hierzu bedarf es analog der Tibiakomponente 1, 51 eines geeigneten Zuflusses für die medizinische Spülflüssigkeit und eines geeigneten Abflusses für die Spülflüssigkeit sowie zumindest eine analog der Tibiakomponente 1, 51 ausgeführte Spülflüssigkeitseintrittsöffnung und zumindest eine Spülflüssigkeitsaustrittsöffnung, die über Leitungen voneinander getrennt sind und mit dem Zufluss oder dem Abfluss verbunden sind. Vorzugsweise werden die Zuflüsse und Abflüsse der Tibiakomponente 1, 51 und der spülbaren Femurkomponente (nicht gezeigt) separat gehalten.

### Bezugszeichenliste

- 1,51: Tibiakomponente
- 2, 52: Femurkomponente
- 3, 53: Lauffläche
- 4, 54: Lauffläche
- 5, 55: Befestigungsfläche
- 6, 56: Befestigungsfläche
- 8, 58: Verbindungsmittel zum Zuführen einer Spülflüssigkeit
- 9, 59: Verbindungsmittel zum Abführen einer Spülflüssigkeit
- 10,60: Spülflüssigkeitsaustrittsöffnung
- 12, 62: Spülflüssigkeitseintrittsöffnung
- 14, 64: Sattel
- 16, 66: Steg
- 17, 67: Steg
- 18, 68: Schlauch
- 19, 69: Schlauch
- 20, 70: Luer-Lock-Adapter
- 21, 71: Luer-Lock-Adapter
- 22, 72: Spülflüssigkeitsaustrittsöffnung
- 24, 74: Spülflüssigkeitseintrittsöffnung
- 26, 76: distaler Vorsprung
- 28, 78: Leitung
- 29, 79: Leitung
- 31, 81: Ventilelement
- 32, 82: Ventilelement
- 33, 83: Verbindungselement
- 34, 84: Verbindungselement
- 35: Gegenbefestigungselement
- 41, 91: Steg
- 42, 92: Befestigungsfläche
- 43, 93: Kondyle
- 44, 94: Kondyle
- 85, 87: Gegenbefestigungselement
- 86, 88: Gegenbefestigungselement
- 95, 98: Spülflüssigkeits-Einlassöffnung
- 96, 97: Spülflüssigkeits-Auslassöffnung

## Patentansprüche

1. Temporäres Kniespacersystem zum zeitweisen Ersetzen eines Kniegelenks, das Kniespacersystem aufweisend
eine Tibiakomponente (1, 51), wobei die Tibiakomponente (1, 51) einen Tibia-Prothesenkörper aufweist, wobei der Tibia-Prothesenkörper zwei Laufflächen (3, 4, 53, 54) auf einer proximalen Seite der Tibiakomponente (1, 51) aufweist und zumindest eine Befestigungsfläche (5, 6, 55, 56) zur Befestigung der Tibiakomponente (1, 51) an einer Tibia auf einer distalen Seite des Tibia-Prothesenkörpers aufweist,
ein erstes röhrenförmiges und flüssigkeitsdurchlässiges Verbindungsmittel (8, 58) zum Zuführen einer medizinischen Spülflüssigkeit in den Tibia-Prothesenkörper,
ein zweites röhrenförmiges und flüssigkeitsdurchlässiges Verbindungsmittel (9, 59) zum Abführen der Spülflüssigkeit aus dem Tibia-Prothesenkörper,
eine Spülflüssigkeits-Einlassöffnung (95, 98) in einer Oberfläche des Tibia-Prothesenkörpers, wobei das erste Verbindungsmittel (8, 58) mit der Spülflüssigkeits-Einlassöffnung (95, 98) flüssigkeitsdurchlässig verbunden oder verbindbar ist,
eine Spülflüssigkeits-Auslassöffnung (96, 97) in der Oberfläche des Tibia-Prothesenkörpers, wobei das zweite Verbindungsmittel (9, 59) mit der Spülflüssigkeits-Auslassöffnung (96, 97) flüssigkeitsdurchlässig verbunden oder verbindbar ist, zumindest eine Spülflüssigkeitsaustrittsöffnung (10, 22, 60, 72) in der Oberfläche des Tibia-Prothesenkörpers, die im Inneren des Tibia-Prothesenkörpers mit der Spülflüssigkeits-Einlassöffnung (95, 98) flüssigkeitsdurchlässig verbunden ist, und zumindest eine Spülflüssigkeitseintrittsöffnung (12, 24, 62, 74) in der Oberfläche des Tibia-Prothesenkörpers, die im Inneren des Prothesenkörpers mit der Spülflüssigkeits-Auslassöffnung (96, 97) flüssigkeitsdurchlässig verbunden ist, wobei
die zumindest eine Spülflüssigkeitsaustrittsöffnung (10, 22, 60, 72) und die zumindest eine Spülflüssigkeitseintrittsöffnung (12, 24, 62, 74) außerhalb der zumindest einen Befestigungsfläche (5, 6, 55, 56) angeordnet sind und wobei
die zumindest eine Spülflüssigkeitsaustrittsöffnung (10, 22, 60, 72) im Inneren des Prothesenkörpers nicht flüssigkeitsdurchlässig mit der Spülflüssigkeits-Auslassöffnung (96, 97) verbunden ist und die zumindest eine Spülflüssigkeitseintrittsöffnung (12, 24, 62, 74) im Inneren des Prothesenkörpers nicht flüssigkeitsdurchlässig mit der Spülflüssigkeits-Einlassöffnung (95, 98) verbunden ist.

2. Kniespacersystem nach Anspruch 1, **dadurch gekennzeichnet, dass**
die zumindest eine Befestigungsfläche (5, 6, 55, 56) begrenzt ist oder
die zumindest eine Befestigungsfläche (5, 6, 55, 56) mit einem umlaufenden und sich aus der Oberfläche des Tibia-Prothesenkörpers erhebenden Steg (16, 17, 66, 67) begrenzt ist, so dass die zumindest eine Befestigungsfläche (5, 6, 55, 56) zur Aufnahme von Knochenzementteig innerhalb des Stegs (16, 17, 66, 67) geeignet ist oder
die zumindest eine Spülflüssigkeitsaustrittsöffnung (10, 22, 60, 72) und die zumindest eine Spülflüssigkeitseintrittsöffnung (12, 24, 62, 74) durch einen umlaufenden und sich aus der Oberfläche des Prothesenkörpers erhebenden Steg (16, 17, 66, 67) von der zumindest einen Befestigungsfläche (5, 6, 55, 56) abgegrenzt sind.

3. Kniespacersystem nach einem der vorangehenden Ansprüche, **dadurch gekennzeichnet, dass**
die Tibiakomponente (1, 51) wenigstens zwei Spülflüssigkeitsaustrittsöffnungen (10, 22, 60, 72) und wenigstens zwei Spülflüssigkeitseintrittsöffnungen (12, 24, 62, 74) in der Oberfläche des Tibia-Prothesenkörpers aufweist, die an unterschiedlichen Seiten des Tibia-Prothesenkörpers angeordnet sind, wobei vorzugsweise eine Spülflüssigkeitsaustrittsöffnung (10, 60) und eine Spülflüssigkeitseintrittsöffnung (12, 62) auf der proximalen Seite des Tibia-Prothesenkörpers angeordnet sind und eine Spülflüssigkeitsaustrittsöffnung (22, 72) und eine Spülflüssigkeitseintrittsöffnung (24, 74) auf der distalen Seite des Tibia-Prothesenkörpers angeordnet sind.

4. Kniespacersystem nach einem der vorangehenden Ansprüche, **dadurch gekennzeichnet, dass**
das erste Verbindungsmittel (8, 58) an der zur Verbindung mit der Spülflüssigkeits-Einlassöffnung (95, 98) abgewandten Seite und das zweite Verbindungmittel an der zur Verbindung mit der Spülflüssigkeits-Auslassöffnung (96, 97) abgewandten Seite jeweils einen Adapter (20, 21, 70, 71) aufweist, insbesondere jeweils ein Luer-Lock-Adapter (20, 21, 70, 71) aufweist, und/oder
die zumindest eine Spülflüssigkeitsaustrittsöffnung (10, 22, 60, 72) und die zumindest eine Spülflüssigkeitseintrittsöffnung (12, 24, 62, 74) voneinander paarweise beabstandet sind, wobei der Abstand zwischen jedem Paar der zumindest einen Spülflüssigkeitsaustrittsöffnung (10, 22, 60, 72) und der zumindest einen Spülflüssigkeitseintrittsöffnung (12, 24, 62, 74) zumindest 5 mm, bevorzugt zumindest 20 mm und besonders bevorzugt zumindest 30 mm beträgt.

5. Kniespacersystem nach einem der vorangehenden Ansprüche, **dadurch gekennzeichnet, dass**
an der Spülflüssigkeits-Einlassöffnung (95, 98) im Inneren des Tibia-Prothesenkörpers oder an der Oberfläche des Tibia-Prothesenkörpers eine selbstdichtende Kupplung angeordnet ist und an der Spülflüssigkeits-Auslassöffnung (96, 97) im Inneren des Tibia-Prothesenkörpers oder an der Oberfläche des Tibia-Prothesenkörpers eine selbstdichtende Kupplung angeordnet ist, wobei das erste Verbindungsmittel (8, 58) lösbar mit der Spülflüssigkeits-Einlassöffnung (95, 98) verbunden oder verbindbar ist und das zweite Verbindungsmittel (9, 59) lösbar mit der Spülflüssigkeits-Auslassöffnung (96, 97) verbunden oder verbindbar ist.

6. Kniespacersystem nach einem der vorangehenden Ansprüche, **dadurch gekennzeichnet, dass**
die Spülflüssigkeits-Einlassöffnung (95) eine erste Spülflüssigkeits-Einlassöffnung (95) ist und die Spülflüssigkeits-Auslassöffnung (96) eine erste Spülflüssigkeits-Auslassöffnung (96) ist, wobei zusätzlich eine zweite Spülflüssigkeits-Einlassöffnung (98) und eine zweite Spülflüssigkeits-Auslassöffnung (97) in der Oberfläche des Tibia-Prothesenkörpers vorgesehen sind, wobei an der ersten Spülflüssigkeits-Einlassöffnung (95), der zweiten Spülflüssigkeits-Einlassöffnung (98), der ersten Spülflüssigkeits-Auslassöffnung (96) und der zweiten Spülflüssigkeits-Auslassöffnung (97) jeweils eine selbstdichtende Kupplung angeordnet ist, wobei das erste Verbindungsmittel (8, 58) flüssigkeitsdicht und lösbar mit der ersten Spülflüssigkeits-Einlassöffnung (95) und mit der zweiten Spülflüssigkeits-Einlassöffnung (98) verbindbar ist und das zweite Verbindungsmittel (9, 59) flüssigkeitsdicht und lösbar mit der ersten Spülflüssigkeits-Auslassöffnung (96) und mit der zweiten Spülflüssigkeits-Auslassöffnung (97) verbindbar ist, wobei
die erste Spülflüssigkeits-Einlassöffnung (95) und die zweite Spülflüssigkeits-Einlassöffnung (98) im Tibia-Prothesenkörper miteinander flüssigkeitsdurchlässig verbunden sind und die erste Spülflüssigkeits-Auslassöffnung (96) und die zweite Spülflüssigkeits-Auslassöffnung (97) im Tibia-Prothesenkörper miteinander flüssigkeitsdurchlässig verbunden sind, wobei bevorzugt
die erste Spülflüssigkeits-Einlassöffnung (95) und die erste Spülflüssigkeits-Auslassöffnung (96) auf einer ersten Seite der Tibiakomponente (1, 51) bezüglich der Sagittalebene angeordnet ist und die zweite Spülflüssigkeits-Einlassöffnung (98) und die zweite Spülflüssigkeits-Auslassöffnung (97) auf einer zweiten Seite der Tibiakomponente (1, 51) bezüglich der Sagittalebene angeordnet ist, und wobei besonders bevorzugt
die erste Spülflüssigkeits-Einlassöffnung (95) und die erste Spülflüssigkeits-Auslassöffnung (96) bezüglich der Sagittalebene spiegelbildlich zur zweiten Spülflüssigkeits-Einlassöffnung (98) und zur zweiten Spülflüssigkeits-Auslassöffnung (97) in der Oberfläche des Tibia-Prothesenkörpers angeordnet sind.

7. Kniespacersystem nach einem der vorangehenden Ansprüche, **dadurch gekennzeichnet, dass**
die Querschnittsfläche der zumindest einen Spülflüssigkeitseintrittsöffnung (12, 24, 62, 74) zumindest genauso groß ist wie die Querschnittsfläche der Spülflüssigkeits-Einlassöffnung (95, 98) und/oder
die Summe der Querschnittsflächen der zumindest einen Spülflüssigkeitsaustrittsöffnung (10, 22, 60, 72) zumindest genauso groß ist wie die Querschnittsfläche der Spülflüssigkeits-Auslassöffnung (96, 97).

8. Kniespacersystem nach einem der vorangehenden Ansprüche, **dadurch gekennzeichnet, dass**
wenigstens eine Spülflüssigkeitsaustrittsöffnung (10, 22, 60, 72) der zumindest einen Spülflüssigkeitsaustrittsöffnung (10, 22, 60, 72) und/oder wenigstens eine Spülflüssigkeitseintrittsöffnung (12, 24, 62, 74) der zumindest einen Spülflüssigkeitseintrittsöffnung (12, 24, 62, 74) zwischen den Laufflächen (3, 4, 53, 54) angeordnet ist oder sind, und/oder
die zumindest eine Spülflüssigkeitsaustrittsöffnung (10, 22, 60, 72) und die zumindest eine Spülflüssigkeitseintrittsöffnung (12, 24, 62, 74) außerhalb den Laufflächen (3, 4, 53, 54) angeordnet sind, wobei vorzugsweise
eine Spülflüssigkeitsaustrittsöffnung (10, 22, 60, 72) der zumindest einen Spülflüssigkeitsaustrittsöffnung (10, 22, 60, 72) und eine Spülflüssigkeitseintrittsöffnung (12, 24, 62, 74) der zumindest einen Spülflüssigkeitseintrittsöffnung (12, 24, 62, 74) neben einer der Laufflächen (3, 4, 53, 54) angeordnet sind, bevorzugt innerhalb von 3 mm neben einer der Laufflächen (3, 4, 53, 54) angeordnet sind.

9. Kniespacersystem nach einem der vorangehenden Ansprüche, **dadurch gekennzeichnet, dass**
in dem ersten Verbindungsmittel (8, 58) oder in der Spülflüssigkeits-Einlassöffnung (95, 98) ein erstes Ventilelement (32, 82) angeordnet ist, das ein Rückfließen der Spülflüssigkeit in das erste Verbindungsmittel (8, 58) verhindert, und/oder
in dem zweiten Verbindungsmittel (9, 59) oder in der Spülflüssigkeits-Auslassöffnung (96, 97) ein zweites Ventilelement (31, 81) angeordnet ist, das ein Rückfließen der Spülflüssigkeit in das zweite Verbindungsmittel (9, 59) verhindert, wobei vorzugsweise das erste und das zweite Ventilelement (31, 32, 81, 82) ausgewählt sind aus einem Rückschlagventil, einem Kugelventil mit Feder, einem Lippenventil, einem Bunsenventil oder einem Plattenventil.

10. Kniespacersystem nach einem der vorangehenden Ansprüche, **dadurch gekennzeichnet, dass**
in einer ersten Leitung (29, 79) innerhalb des Tibia-Prothesenkörpers, die die zumindest eine Spülflüssigkeitseintrittsöffnung (12, 24, 62, 74) mit der Spülflüssigkeits-Auslassöffnung (96, 97) flüssigkeitsdurchlässig verbindet, ein erstes Ventil (31, 81) angeordnet ist, das nur durch Anlegen eines Unterdrucks an der Spülflüssigkeits-Auslassöffnung (96, 97) zu öffnen ist und dass ein Zurückfließen der Spülflüssigkeit in die erste Leitung (29, 79) verhindert, und/oder
in einer zweiten Leitung (28, 78) innerhalb des Tibia-Prothesenkörpers, die die zumindest eine Spülflüssigkeitsaustrittsöffnung (10, 22, 60, 72) mit der Spülflüssigkeits-Einlassöffnung (95, 98) flüssigkeitsdurchlässig verbindet, ein zweites Ventil (32, 82) angeordnet ist, das nur durch Anlegen eines Unterdrucks an der Spülflüssigkeits-Einlassöffnung (95, 98) zu öffnen ist und dass ein Zurückfließen der Spülflüssigkeit in die zweite Leitung (28, 78) verhindert.

11. Kniespacersystem nach einem der vorangehenden Ansprüche, **dadurch gekennzeichnet, dass**
in dem Tibia-Prothesenkörper die Spülflüssigkeits-Einlassöffnung (95, 98), die Spülflüssigkeits-Auslassöffnung (96, 97), die zumindest eine Spülflüssigkeitsaustrittsöffnung (10, 22, 60, 72) und die zumindest eine Spülflüssigkeitseintrittsöffnung (12, 24, 62, 74) und die flüssigkeitsdurchlässigen Verbindungen ausgebildet sind, wobei
der Tibia-Prothesenkörper vorzugsweise aus Kunststoff, Metall, Keramik, Glaskeramik, Knochenzement oder einer Kombination daraus gefertigt ist.

12. Kniespacersystem nach einem der vorangehenden Ansprüche, **dadurch gekennzeichnet, dass**
die Spülflüssigkeits-Einlassöffnung (95, 98) und die Spülflüssigkeits-Auslassöffnung (96, 97) in einer seitlichen Oberfläche des Tibia-Prothesenkörpers angeordnet sind oder an einer lateralen seitlichen Oberfläche des Tibia-Prothesenkörpers angeordnet sind und
zumindest eine erste Spülflüssigkeitsaustrittsöffnung (10, 22, 60, 72) der zumindest einen Spülflüssigkeitsaustrittsöffnung (10, 22, 60, 72) auf der proximalen Seite in der Oberfläche des Tibia-Prothesenkörpers angeordnet ist und zumindest eine zweite Spülflüssigkeitsaustrittsöffnung (10, 22, 60, 72) der zumindest einen Spülflüssigkeitsaustrittsöffnung (10, 22, 60, 72) auf der distalen Seite in der Oberfläche des Tibia-Prothesenkörpers angeordnet ist und
zumindest eine erste Spülflüssigkeitseintrittsöffnung (12, 24, 62, 74) der zumindest einen Spülflüssigkeitseintrittsöffnung (12, 24, 62, 74) auf der proximalen Seite in der Oberfläche des Tibia-Prothesenkörpers angeordnet ist und zumindest eine zweite Spülflüssigkeitseintrittsöffnung (12, 24, 62, 74) der zumindest einen Spülflüssigkeitseintrittsöffnung (12, 24, 62, 74) auf der distalen Seite in der Oberfläche des Tibia-Prothesenkörpers angeordnet ist.

13. Kniespacersystem nach einem der vorangehenden Ansprüche, **dadurch gekennzeichnet, dass**
das Kniespacersystem eine Femurkomponente (2, 52) aufweist, wobei die Femurkomponente (2, 52) auf einer distalen Seite zwei Kondylen (43, 44, 93, 94) aufweist.

14. Kniespacersystem nach Anspruch 13, **dadurch gekennzeichnet, dass**
die Femurkomponente (2, 52) einen Femur-Prothesenkörper aufweist, wobei der Femur-Prothesenkörper zumindest eine Befestigungsfläche (42, 92) zur Befestigung der Femurkomponente (2, 52) mit einem Knochenzement an einem Femur auf einer proximalen Seite des Femur-Prothesenkörpers aufweist, die Femurkomponente (2, 52) ferner aufweisend
ein drittes röhrenförmiges und flüssigkeitsdurchlässiges Verbindungsmittel zum Zuführen einer medizinischen Spülflüssigkeit in den Femur-Prothesenkörper,
ein viertes röhrenförmiges und flüssigkeitsdurchlässiges Verbindungsmittel zum Abführen der Spülflüssigkeit aus dem Femur-Prothesenkörper,
eine Spülflüssigkeits-Einlassöffnung in einer Oberfläche des Femur-Prothesenkörpers, wobei das dritte Verbindungsmittel mit der Spülflüssigkeits-Einlassöffnung des Femur-Prothesenkörpers flüssigkeitsdurchlässig verbunden oder verbindbar ist,
eine Spülflüssigkeits-Auslassöffnung in der Oberfläche des Femur-Prothesenkörpers, wobei das vierte Verbindungsmittel mit der Spülflüssigkeits-Auslassöffnung des Femur-Prothesenkörpers flüssigkeitsdurchlässig verbunden oder verbindbar ist, zumindest eine Spülflüssigkeitsaustrittsöffnung in der Oberfläche des Femur-Prothesenkörpers, die im Inneren des Femur-Prothesenkörpers mit der Spülflüssigkeits-Einlassöffnung des Femur-Prothesenkörpers flüssigkeitsdurchlässig verbunden ist, und
zumindest eine Spülflüssigkeitseintrittsöffnung in der Oberfläche des Femur-Prothesenkörpers, die im Inneren des Femur-Prothesenkörpers mit der Spülflüssigkeits-Auslassöffnung des Femur-Prothesenkörpers flüssigkeitsdurchlässig verbunden ist, wobei
die zumindest eine Spülflüssigkeitsaustrittsöffnung des Femur-Prothesenkörpers und die zumindest eine Spülflüssigkeitseintrittsöffnung des Femur-Prothesenkörpers außerhalb der zumindest einen Befestigungsfläche (42, 92) des Femur-Prothesenkörpers angeordnet sind, und wobei
die zumindest eine Spülflüssigkeitsaustrittsöffnung des Femur-Prothesenkörpers und die zumindest eine Spülflüssigkeitseintrittsöffnung des Femur-Prothesenkörpers außerhalb der Kondylen (43, 44, 93, 94) der Femurkomponente (2, 52) angeordnet sind.

15. Kniespacersystem nach einem der vorangehenden Ansprüche, **dadurch gekennzeichnet, dass**
wenigstens eine Spülflüssigkeitsaustrittsöffnung (10, 22, 60, 72) der zumindest einen Spülflüssigkeitsaustrittsöffnung (10, 22, 60, 72) oder wenigstens eine Spülflüssigkeitseintrittsöffnung (12, 24, 62, 74) der zumindest einen Spülflüssigkeitseintrittsöffnung (12, 24, 62, 74) an einer distalen Seite eines sich in distaler Richtung erstreckenden Vorsprungs (26, 76) zwischen zwei Befestigungsflächen (5, 6, 55, 56) der Tibiakomponente (1, 51) angeordnet ist.

## Claims

1. A temporary knee spacer system for the temporary replacement of a knee joint, the knee spacer system comprising
a tibia component (1, 51), wherein the tibia component (1, 51) has a tibia prosthetic body, wherein the tibia prosthetic body has two running surfaces (3, 4, 53, 54) on a proximal side of the tibia component (1, 51) and at least one mounting surface (5, 6, 55, 56) for mounting the tibia component (1, 51) on a tibia on a distal side of the tibia prosthetic body,
a first tubular and fluid-permeable connecting means (8, 58) for feeding a medical irrigation fluid into the tibia prosthetic body,
a second tubular and fluid-permeable connecting means (9, 59) for draining a medical irrigation fluid from the tibia prosthetic body,
an irrigation fluid inlet opening (95, 98) in a surface of the tibia prosthetic body, wherein the first connecting means (8, 58) is connected or connectable in a fluid-permeable manner to the irrigation fluid inlet opening (95, 98),
an irrigation fluid outlet opening (96, 97) in the surface of the tibia prosthetic body, wherein the second connecting means (9, 59) is connected or connectable in a fluid-permeable manner to the irrigation fluid outlet opening (96, 97),
at least one irrigation fluid exit opening (10, 22, 60, 72) in the surface of the tibia prosthetic body, which is connected in the interior of the tibia prosthetic body with the irrigation fluid inlet opening (95, 98) in a fluid-permeable manner, and
at least one irrigation fluid entry opening (12, 24, 62, 74) in the surface of the tibia prosthetic body, which is connected in the interior of the tibia prosthetic body with the irrigation fluid outlet opening (96, 97) in a fluid-permeable manner, wherein
the at least one irrigation fluid exit opening (10, 22, 60, 72) and the at least one irrigation fluid entry opening (12, 24, 62, 74) are arranged outside the at least one mounting surface (5, 6, 55, 56), and wherein
the at least one irrigation fluid exit opening (10, 22, 60, 72) in the interior of the prosthetic body is not connected in a fluid-permeable manner with the irrigation fluid outlet opening (96, 97) and the at least one irrigation fluid entry opening (12, 24, 62, 74) in the interior of the prosthetic body is not connected with the irrigation fluid inlet opening (95, 98) in a fluid-permeable manner.

2. The knee spacer system according to Claim 1, **characterized in that** the at least one mounting surface (5, 6, 55, 56) is bound, or
the at least one mounting surface (5, 6, 55, 56) is bound by a surrounding ridge (16, 17, 66, 67) that rises from the surface of the tibia prosthetic body, so that the at least one mounting surface (5, 6, 55, 56) is suitable for holding bone cement paste within the ridge (16, 17, 66, 67) or
the at least one irrigation liquid exit opening (10, 22, 60, 72) and the at least one irrigation liquid entry opening (12, 24, 62, 74) are separated from the at least one mounting surface (5, 6, 55, 56) by a surrounding ridge (16, 17, 66, 67) that rises from the surface of the prosthetic body.

3. The knee spacer system according to any one of the preceding claims, **characterized in that**
the tibia component (1, 51) has at least two irrigation fluid exit openings (10, 22, 60, 72) and at least two irrigation fluid entry openings (12, 24, 62, 74) in the surface of the tibia prosthetic body, which are arranged on different sides of the tibia prosthetic body, herein preferably
an irrigation fluid exit opening (10, 60) and an irrigation fluid entry opening (12, 62) are arranged on the proximal side of the tibia prosthetic body and an irrigation fluid exit opening (22, 72) and an irrigation fluid entry opening (24, 74) are arranged on the distal side of the tibia prosthetic body.

4. The knee spacer system according to any one of the preceding claims, **characterized in that**
the first connecting means (8, 58) on the side facing away from the connection with the irrigation fluid inlet opening (95, 98) and the second connecting means on the side facing away from the connection with the irrigation fluid outlet opening (96, 97) each has one adapter (20, 21, 70, 71), in particular each has a Luer lock adapter (20, 21, 70, 71), and/or
the at least one irrigation fluid exit opening (10, 22, 62, 72) and the at least one irrigation fluid entry opening (12, 24, 62, 74) are spaced apart from each other in pairs, wherein the space between each pair of the at least one irrigation fluid exit opening (10, 22, 60, 72) and the at least one irrigation fluid entry opening (12, 24, 62, 74) is at least 5 mm, preferably at least 20 mm and particularly preferably, at least 30 mm.

5. The knee spacer system according to any one of the preceding claims, **characterized in that**
at the irrigation fluid inlet opening (95, 98) in the interior of the tibia prosthetic body or on the surface of the tibia prosthetic body, a self-sealing coupling is arranged, and at the irrigation fluid outlet opening (96, 97) in the interior of the tibia prosthetic body or on the surface of the tibia prosthetic body, a self-sealing coupling is arranged, wherein the first connecting means (8, 58) is detachably connected or connectable with the irrigation fluid inlet opening (95, 98) and the second connecting means (9, 59) is detachably connected or connectable with the irrigation fluid outlet opening (96, 97).

6. The knee spacer system according to any one of the preceding claims, **characterized in that**
the irrigation liquid inlet opening (95) is a first irrigation liquid inlet opening (95), and the irrigation liquid outlet opening (96) is a first irrigation liquid outlet opening (96), wherein additionally, a second irrigation liquid inlet opening (98) and a second irrigation liquid outlet opening (97) are provided in the surface of the tibia prosthetic body, wherein at each of the first irrigation liquid inlet opening (95), the second irrigation liquid inlet opening (98), the first irrigation liquid outlet opening (96) and the second irrigation liquid outlet opening (97), one self-sealing coupling is arranged, wherein the first connecting means (8, 58) is connectable in a fluid-tight and detachable manner with the first irrigation liquid inlet opening (95) and with the second irrigation liquid inlet opening (98), and the second connecting means (9, 59) is connectable in a fluid-tight and detachable manner with the first irrigation liquid outlet opening (96) and with the second irrigation liquid outlet opening (97), wherein
the first irrigation liquid inlet opening (95) and the second irrigation liquid inlet opening (98) are connected in a fluid-permeable manner with each other in the tibia prosthetic body, and the first irrigation liquid outlet opening (96) and the second irrigation liquid outlet opening (97) are connected in a fluid-permeable manner with each other in the tibia prosthetic body, wherein preferably
the first irrigation liquid inlet opening (95) and the first irrigation liquid outlet opening (96) are arranged on a first side of the tibia component (1, 51) in relation to a sagittal plane, and the second irrigation liquid inlet opening (98) and the second irrigation liquid outlet opening (97) is arranged on a second side of the tibia component (1, 51) in relation to the sagittal plane, and wherein particularly preferably
the first irrigation liquid inlet opening (95) and the first irrigation liquid outlet opening (96) are arranged in relation to the sagittal plane in a mirror-inverted manner to the second irrigation liquid inlet opening (98) and to the second irrigation liquid outlet opening (97) in the surface of the tibia prosthetic body.

7. The knee spacer system according to any one of the preceding claims, **characterized in that**
a cross-sectional area of the at least one irrigation liquid entry opening (12, 24, 62, 74) is at least just as large as a cross-sectional area of the irrigation liquid inlet opening (95, 98), and/or
the total of cross-sectional areas of the at least one irrigation liquid exit opening (10, 22, 60, 72) is at least just as large as a cross-sectional area of the irrigation liquid outlet opening (96, 97).

8. The knee spacer system according to any one of the preceding claims, **characterized in that**
at least one irrigation fluid exit opening (10, 22, 60, 72) of the at least one irrigation fluid exit opening (10, 22, 60, 72) and/or at least one irrigation fluid entry opening (12, 24, 62, 74) of the least one irrigation fluid entry opening (12, 24, 62, 74) is or are arranged between the running surfaces (3, 4, 53, 54), and/or
the at least one irrigation fluid exit opening (10, 22, 60, 72) and the at least one irrigation fluid entry opening (12, 24, 62, 74) are arranged outside the running surfaces (3, 4, 53, 54), wherein preferably
one irrigation fluid exit opening (10, 22, 60, 72) of the at least one irrigation fluid exit opening (10, 22, 60, 72) and one irrigation fluid entry opening (12, 24, 62, 74) of the least one irrigation fluid entry opening (12, 24, 62, 74) are arranged adjacent to one of the running surfaces (3, 4, 53, 54), preferably within 3 mm adjacent to one of the running surfaces (3, 4, 53, 54).

9. The knee spacer system according to any one of the preceding claims, **characterized in that**
in the first connecting means (8, 58) or in the irrigation fluid inlet opening (95, 98), a first valve element (32, 82) is arranged that prevents a return flow of the irrigation fluid into the first connecting means (8, 58), and/or
in the second connecting means (9, 59) or in the irrigation fluid outlet opening (96, 97), a second valve element (31, 81) is arranged that prevents a return flow of the irrigation fluid into the second connecting means (9, 59), wherein preferably
the first and second valve element (31, 32, 81, 82) are selected from a non-return valve, a ball valve with spring, a lip valve, a Bunsen valve or a plate valve.

10. The knee spacer system according to any one of the preceding claims, **characterized in that**
in a first line (29, 79) within the tibia prosthetic body, which connects the at least one irrigation fluid entry opening (12, 24, 62, 74) with the irrigation fluid outlet opening (96, 97) in a fluid-permeable manner, a first valve (31, 81) is arranged which can only be opened by applying negative pressure to the irrigation fluid outlet opening (96, 97), and which prevents a return flow of the irrigation fluid into the first line (29, 79), and/or
in a second line (28, 78) within the tibia prosthetic body, which connects the at least one irrigation fluid exit opening (10, 22, 60, 72) with the irrigation fluid inlet opening (95, 98) in a fluid-permeable manner, a second valve (32, 82) is arranged which can only be opened by applying negative pressure to the irrigation fluid inlet opening (95, 98), and which prevents a return flow of the irrigation fluid into the second line (28, 78).

11. The knee spacer system according to any one of the preceding claims, **characterized in that**
in the tibia prosthetic body, the irrigation fluid inlet opening (95, 98), the irrigation fluid outlet opening (96, 97), the at least one irrigation fluid exit opening (10, 22, 60, 72) and the at least one irrigation fluid entry opening (12, 24, 62, 74) and the fluid-permeable connections are formed, wherein
the tibia prosthetic body is preferably made of plastic, metal, ceramic, glass ceramic, bone cement or a combination of these.

12. The knee spacer system according to any one of the preceding claims, **characterized in that**
the irrigation fluid inlet opening (95, 98) and the irrigation fluid outlet opening (96, 97) are arranged in a side surface of the tibia prosthetic body or on a lateral side surface of the tibia prosthetic body, and
at least one first irrigation fluid exit opening (10, 22, 60, 72) of the at least one irrigation fluid exit opening (10, 22, 60, 72) is arranged on the proximal side in the surface of the tibia prosthetic body, and at least one second irrigation fluid exit opening (10, 22, 60, 72) of the at least one irrigation fluid exit opening (10, 22, 60, 72) is arranged on the distal side in the surface of the tibia prosthetic body, and
at least one first irrigation fluid entry opening (12, 24, 62, 74) of the at least one irrigation fluid entry opening (12, 24, 62, 74) is arranged on the proximal side in the surface of the tibia prosthetic body, and at least one second irrigation fluid entry opening (12, 24, 62, 74) of the at least one irrigation fluid entry opening (12, 24, 62, 74) is arranged on the distal side in the surface of the tibia prosthetic body.

13. The knee spacer system according to any one of the preceding claims, **characterized in that**
the knee spacer system has a femur component (2, 52), wherein the femur component (2, 52) has two condyles (43, 44, 93, 94) on one distal side.

14. The knee spacer system according to Claim 13, **characterized in that**
the femur component (2, 52) has a femur prosthetic body, wherein the femur prosthetic body has at least one mounting surface (42, 92) for mounting the femur component (2, 52) with a bone cement to a femur on one proximal side of the femur prosthetic body, the femur component (2, 52) further having
a third tubular and fluid-permeable connecting means for feeding a medical irrigation fluid into the femur prosthetic body,
a fourth tubular and fluid-permeable connecting means for draining a medical irrigation fluid from the femur prosthetic body,
an irrigation fluid inlet opening in a surface of the femur prosthetic body, wherein the third connecting means is connected or connectable in a fluid-permeable manner to the irrigation fluid inlet opening of the femur prosthetic body,
an irrigation fluid outlet opening in a surface of the femur prosthetic body, wherein the fourth connecting means is connected or connectable in a fluid-permeable manner to the irrigation fluid outlet opening of the femur prosthetic body,
at least one irrigation fluid exit opening in the surface of the femur prosthetic body, which is connected in the interior of the femur prosthetic body with the irrigation fluid inlet opening of the femur prosthetic body in a fluid-permeable manner, and
at least one irrigation fluid entry opening in the surface of the femur prosthetic body, which is connected in the interior of the femur prosthetic body with the irrigation fluid outlet opening of the femur prosthetic body in a fluid-permeable manner, wherein
the at least one irrigation fluid exit opening of the femur prosthetic body and the at least one irrigation fluid entry opening of the femur prosthetic body are arranged outside the at least one mounting surface (42, 92) of the femur prosthetic body, and wherein
the at least one irrigation fluid exit opening of the femur prosthetic body and the at least one irrigation fluid entry opening of the femur prosthetic body are arranged outside the condyles (43, 44, 93, 94) of the femur prosthetic body.

15. The knee spacer system according to any one of the preceding claims, **characterized in that**
at least one irrigation fluid exit opening (10, 22, 60, 72) of the at least one irrigation fluid exit opening (10, 22, 60, 72) or at least one irrigation fluid entry opening (12, 24, 62, 74) of the least one irrigation fluid entry opening (12, 24, 62, 74) is arranged on a distal side of a protrusion (26, 76) extending in the distal direction between two mounting surfaces (5, 6, 55, 56) of the tibia component (1, 51).

## Revendications

1. Système d'espaceur de genou temporaire permettant de remplacer momentanément une articulation de genou, le système d'espaceur de genou présentant
un composant tibial (1, 51), où le composant tibial (1, 51) présente un corps de prothèse de tibia, où le corps de prothèse de tibia présente deux surfaces de glissement (3, 4, 53, 54) sur un côté proximal du composant tibial (1, 51) et au moins une surface de fixation (5, 6, 55, 56) pour la fixation du composant tibial (1, 51) sur un tibia sur un côté distal du corps de prothèse de tibia,
un premier moyen de liaison (8, 58) en forme de tube et laissant passer du liquide pour l'approvisionnement d'un liquide de lavage médicinal dans le corps de prothèse de tibia,
un deuxième moyen de liaison (9, 59) en forme de tube et laissant passer du liquide pour l'évacuation du liquide de lavage hors du corps de prothèse de tibia,
un orifice d'entrée de liquide de lavage (95, 98) dans une surface du corps de prothèse de tibia, où le premier moyen de liaison (8, 58) est relié ou peut être relié avec l'orifice d'entrée de liquide de lavage (95, 98) en laissant passer le liquide,
un orifice de sortie de liquide de lavage (96, 97) dans la surface du corps de prothèse de tibia, où le deuxième moyen de liaison (9, 59) est relié ou peut être relié avec l'orifice de sortie de liquide de lavage (96, 97) en laissant passer le liquide,
au moins un orifice d'évacuation de liquide de lavage (10, 22, 60, 72) dans la surface du corps de prothèse de tibia qui est relié à l'intérieur du corps de prothèse de tibia avec l'orifice d'entrée de liquide de lavage (95, 98) en laissant passer le liquide, et
au moins un orifice d'alimentation de liquide de lavage (12, 24, 62, 74) dans la surface du corps de prothèse de tibia qui est relié à l'intérieur du corps de prothèse de tibia avec l'orifice de sortie de liquide de lavage (96, 97) en laissant passer le liquide, où l'au moins un orifice d'évacuation de liquide de lavage (10, 22, 60, 72) et l'au moins un orifice d'alimentation de liquide de lavage (12, 24, 62, 74) sont disposés à l'extérieur de l'au moins une surface de fixation (5, 6, 55, 56), et où
l'au moins un orifice d'évacuation de liquide de lavage (10, 22, 60, 72) est relié avec l'orifice de sortie de liquide de lavage (96, 97) à l'intérieur du corps de prothèse en ne laissant pas passer le liquide, et l'au moins un orifice d'alimentation de liquide de lavage (12, 24, 62, 74) est relié avec l'orifice d'entrée de liquide de lavage (95, 98) à l'intérieur du corps de prothèse en ne laissant pas passer le liquide.

2. Système d'espaceur de genou selon la revendication 1, **caractérisé en ce que** l'au moins une surface de fixation (5, 6, 55, 56) est délimitée ou
l'au moins une surface de fixation (5, 6, 55, 56) est délimitée avec un étai (16, 17, 66, 67) périphérique et s'élevant à partir de la surface du corps de prothèse de tibia, de sorte que l'au moins une surface de fixation (5, 6, 55, 56) est appropriée pour la réception de pâte de ciment osseux à l'intérieur de l'étai (16, 17, 66, 67), ou
l'au moins un orifice d'évacuation de liquide de lavage (10, 22, 60, 72) et l'au moins un orifice d'alimentation de liquide de lavage (12, 24, 62, 74) sont délimités par un étai (16, 17, 66, 67) périphérique et s'élevant à partir de la surface du corps de prothèse par l'au moins une surface de fixation (5, 6, 55, 56).

3. Système d'espaceur de genou selon l'une des revendications précédentes, **caractérisé en ce que**
le composant tibial (1, 51) présente au moins deux orifices d'évacuation de liquide de lavage (10, 22, 60, 72) et au moins deux orifices d'alimentation de liquide de lavage (12, 24, 62, 74) dans la surface du corps de prothèse de tibia, qui sont disposés sur des côtés différents du corps de prothèse de tibia, où, de préférence,
un orifice d'évacuation de liquide de lavage (10, 60) et un orifice d'alimentation de liquide de lavage (12, 62) sont disposés sur le côté proximal du corps de prothèse de tibia et un orifice d'évacuation de liquide de lavage (22, 72) et un orifice d'alimentation de liquide de lavage (24, 74) sont disposés sur l'extrémité distale du corps de prothèse de tibia.

4. Système d'espaceur de genou selon l'une des revendications précédentes, **caractérisé en ce que**
le premier moyen de liaison (8, 58) présente, au niveau du côté opposé à la liaison avec l'orifice d'entrée de liquide de lavage (95, 98), et le deuxième moyen de liaison présente, au niveau du côté opposé à la liaison avec l'orifice de sortie de liquide de lavage (96, 97), respectivement un adaptateur (20, 21, 70, 71), notamment, respectivement un adaptateur (20, 21, 70, 71) Luer-Lock, et/ou
l'au moins un orifice d'évacuation de liquide de lavage (10, 22, 60, 72) et l'au moins un orifice d'alimentation de liquide de lavage (12, 24, 62, 74) sont espacés par paires les uns des autres, où la distance entre chaque paire de l'au moins un orifice d'évacuation de liquide de lavage (10, 22, 60, 72) et l'au moins un orifice d'alimentation de liquide de lavage (12, 24, 62, 74) est d'au moins 5 mm, de préférence, d'au moins 20 mm et de manière particulièrement préférée, d'au moins 30 mm.

5. Système d'espaceur de genou selon l'une des revendications précédentes, **caractérisé en ce**
**qu'**un accouplement auto-obturant est disposé sur l'orifice d'entrée de liquide de lavage (95, 98) à l'intérieur du corps de prothèse de tibia ou sur la surface du corps de prothèse de tibia, et un accouplement auto-obturant est disposé sur l'orifice de sortie de liquide de lavage (96, 97) à l'intérieur du corps de prothèse de tibia ou sur la surface du corps de prothèse de tibia, où le premier moyen de liaison (8, 58) est relié ou peut être relié de façon amovible avec l'orifice d'entrée de liquide de lavage (95, 98) et le deuxième moyen de liaison (9, 59) est relié ou peut être relié de façon amovible avec l'orifice de sortie de liquide de lavage (96, 97).

6. Système d'espaceur de genou selon l'une des revendications précédentes, **caractérisé en ce que**
l'orifice d'entrée de liquide de lavage (95) est un premier orifice d'entrée de liquide de lavage (95) et l'orifice de sortie de liquide de lavage (96) est un premier orifice de sortie de liquide de lavage (96), où, en outre, un deuxième orifice d'entrée de liquide de lavage (98) et un deuxième orifice de sortie de liquide de lavage (97) sont prévus dans la surface du corps de prothèse de tibia, où, au niveau du premier orifice d'entrée de liquide de lavage (95), du deuxième orifice d'entrée de liquide de lavage (98), du premier orifice d'évacuation de liquide de lavage (96) et du deuxième orifice de sortie de liquide de lavage (97) il y a respectivement un accouplement auto-obturant, où le premier moyen de liaison (8, 58) ne laisse pas passer les liquides et peut être relié de façon amovible avec le premier orifice d'entrée de liquide de lavage (95) et avec le deuxième orifice d'entrée de liquide de lavage (98) et le deuxième moyen de liaison (9, 59) peut être relié de manière à ne pas laisser passer de fluide et amovible avec le premier orifice de sortie de liquide de lavage (96) et avec le deuxième orifice de sortie de liquide de lavage (97), où
le premier orifice d'entrée de liquide de lavage (95) et le deuxième orifice d'entrée de liquide de lavage (98) sont reliés dans le corps de prothèse de tibia l'un à l'autre en laissant passer du liquide et le premier orifice de sortie de liquide de lavage (96) et le deuxième orifice de sortie de liquide de lavage (97) sont reliés dans le corps de prothèse de tibia l'un à l'autre en laissant passer le liquide, où, de préférence,
le premier orifice d'entrée de liquide de lavage (95) et le premier orifice de sortie de liquide de lavage (96) sont disposés sur un premier côté du composant tibial (1, 51) par rapport au plan sagittal et le deuxième orifice d'entrée de liquide de lavage (98) et le deuxième orifice de sortie de liquide de lavage (97) sont disposés sur un deuxième côté du composant tibial (1, 51) par rapport au plan sagittal, et où, de manière particulièrement préférée,
le premier orifice d'entrée de liquide de lavage (95) et le premier orifice de sortie de liquide de lavage (96) sont disposés, par rapport au plan sagittal, de manière symétrique en miroir par rapport au deuxième orifice d'entrée de liquide de lavage (98) et au deuxième orifice de sortie de liquide de lavage (97) dans la surface du corps de prothèse de tibia.

7. Système d'espaceur de genou selon l'une des revendications précédentes, **caractérisé en ce que**
la surface de la section transversale de l'au moins un orifice d'alimentation de liquide de lavage (12, 24, 62, 74) est au moins aussi grande que la surface de la section transversale de l'orifice d'entrée de liquide de lavage (95, 98), et/ou
la somme des surfaces des sections transversales de l'au moins un orifice d'évacuation de liquide de lavage (10, 22, 60, 72) est au moins aussi grande que la surface de la section transversale de l'orifice de sortie de liquide de lavage (96, 97).

8. Système d'espaceur de genou selon l'une des revendications précédentes, **caractérisé en ce**
**qu'**au moins un orifice d'évacuation de liquide de lavage (10, 22, 60, 72) parmi l'au moins un orifice d'évacuation de liquide de lavage (10, 22, 60, 72) et/ou au moins un orifice d'alimentation de liquide de lavage (12, 24, 62, 74) parmi l'au moins un orifice d'alimentation de liquide de lavage (12, 24, 62, 74) est disposé ou sont disposés entre les surfaces de glissement (3, 4, 53, 54), et/ou
l'au moins un orifice d'évacuation de liquide de lavage (10, 22, 60, 72) et l'au moins un orifice d'alimentation de liquide de lavage (12, 24, 62, 74) sont disposés à l'extérieur des surfaces de glissement (3, 4, 53, 54), où, de préférence
un orifice d'évacuation de liquide de lavage (10, 22, 60, 72) parmi l'au moins un orifice d'évacuation de liquide de lavage (10, 22, 60, 72) et un orifice d'alimentation de liquide de lavage (12, 24, 62, 74) parmi l'au moins un orifice d'alimentation de liquide de lavage (12, 24, 62, 74) sont disposés à côté d'une des surfaces de glissement (3, 4, 53, 54), sont disposés de préférence à moins de 3 mm à côté d'une des surfaces de glissement (3, 4, 53, 54).

9. Système d'espaceur de genou selon l'une des revendications précédentes, **caractérisé en ce**
**qu'**un premier élément de type vanne (32, 82) est disposé dans le premier moyen de liaison (8, 58) ou dans l'orifice d'entrée de liquide de lavage (95, 98), qui empêche le retour du liquide de lavage dans le premier moyen de liaison (8, 58), et/ou
**qu'**un deuxième élément de type vanne (31, 81) est disposé dans le deuxième moyen de liaison -9, 59) ou dans l'orifice de sortie de liquide de lavage (96, 97) qui empêche un retour du liquide de lavage dans le deuxième moyen de liaison (9, 59), où, de préférence les premier et deuxième éléments de type vanne (31, 32, 81, 82) sont choisis parmi un clapet anti-retour, un soupape à bille avec ressort, un clapet à lèvre, une valve Bunsen ou un clapet à disque.

10. Système d'espaceur de genou selon l'une des revendications précédentes, **caractérisé en ce que**,
dans une première conduite (29, 79) à l'intérieur du corps de prothèse de tibia, qui relie l'au moins un orifice d'alimentation de liquide de lavage (12, 24, 62, 74) avec l'orifice de sortie de liquide de lavage (96, 97) en laissant passer du liquide, une première vanne (31, 81) est disposée, qui ne peut être ouverte que par l'application d'une pression négative à l'orifice de sortie de liquide de lavage (96, 97) et qui empêche un retour du liquide de lavage dans la première conduite (29, 79), et/ou
dans une deuxième conduite (28, 78) à l'intérieur du corps de prothèse de tibia, qui relie l'au moins un orifice d'évacuation de liquide de lavage (10, 22, 60, 72) avec l'orifice d'entrée de liquide de lavage (95, 98) en laissant passer du liquide, une deuxième vanne (32, 82) est disposée qui ne peut être ouverte que par l'application d'une pression négative à l'orifice d'entrée de liquide de lavage (95, 98) et qui empêche un retour du liquide de lavage dans la deuxième conduite (28, 78).

11. Système d'espaceur de genou selon l'une des revendications précédentes, **caractérisé en ce que**
l'orifice d'entrée de liquide de lavage (95, 98), l'orifice de sortie de liquide de lavage (96, 97), l'au moins un orifice d'évacuation de liquide de lavage (10, 22, 60, 72) et l'au moins un orifice d'alimentation de liquide de lavage (12, 24,62, 74) et les liaisons laissant passer le liquide sont conçus dans le corps de prothèse de tibia, où
le corps de prothèse de tibia est fabriqué à base de matière plastique, de métal, de céramique, de vitrocéramique, de ciment osseux ou d'une combinaison de ceux-ci.

12. Système d'espaceur de genou selon l'une des revendications précédentes, **caractérisé en ce que**,
l'orifice d'entrée de liquide de lavage (95, 98) et l'orifice de sortie de liquide de lavage (96, 97) sont disposés sur une surface d'un côté du corps de prothèse de tibia ou sont disposés sur une surface d'un côté latéral du corps de prothèse de tibia, et
au moins un premier orifice d'évacuation de liquide de lavage (10, 22, 60, 72) parmi l'au moins un orifice d'évacuation de liquide de lavage (10, 22, 60, 72) est disposé sur le côté proximal dans la surface du corps de prothèse de tibia, et au moins un deuxième orifice d'évacuation de liquide de lavage (10, 22, 60, 72) parmi l'au moins un orifice d'évacuation de liquide de lavage (10, 22, 60, 72) est disposé sur le côté distal dans la surface du corps de prothèse de tibia, et
au moins un premier orifice d'alimentation de liquide de lavage (12, 24, 62, 74) parmi l'au moins un orifice d'alimentation de liquide de lavage (12, 24, 62, 74) est disposé sur le côté proximal dans la surface du corps de prothèse de tibia, et au moins un deuxième orifice d'alimentation de liquide de lavage (12, 24, 62, 74) parmi l'au moins un orifice d'alimentation de liquide de lavage (12, 24, 62, 74) est disposé sur le côté distal dans la surface du corps de prothèse de tibia.

13. Système d'espaceur de genou selon l'une des revendications précédentes, **caractérisé en ce que**
le système d'espaceur de genou présente un composant fémoral (2, 52) où le composant fémoral (2, 52) présente deux condyles (43, 44, 93, 94) sur le côté distal.

14. Système d'espaceur de genou selon la revendication 13, **caractérisé en ce que**
le composant fémoral (2, 52) présente un corps de prothèse de fémur, où le corps de prothèse de fémur présente au moins une surface de fixation (42, 92) pour la fixation du composant fémoral (2, 52) avec un ciment osseux à un fémur sur un côté proximal du corps de prothèse de fémur, le composant fémoral (2, 52) présentant en outre
un troisième moyen de liaison en forme de tube et laissant passer le liquide pour l'approvisionnement d'un liquide de lavage médicinal dans le corps de prothèse de fémur,
un quatrième moyen de liaison en forme de tube et laissant passer le liquide pour l'évacuation d'un liquide de lavage médicinal hors du corps de prothèse de fémur,
un orifice d'entrée de liquide de lavage dans une surface du corps de prothèse de fémur, où le troisième moyen de liaison est relié ou peut être relié avec l'orifice d'entrée de liquide de lavage du corps de prothèse de fémur en laissant passer le liquide,
un orifice de sortie de liquide de lavage dans la surface du corps de prothèse de fémur, où le quatrième moyen de liaison est relié ou peut être relié avec l'orifice de sortie de liquide de lavage du corps de prothèse de fémur en laissant passer le liquide,
au moins un orifice d'évacuation de liquide de lavage dans la surface du corps de prothèse de fémur qui est relié à l'intérieur du corps de prothèse de fémur avec l'orifice d'entrée de liquide de lavage du corps de prothèse de fémur en laissant passer le liquide, et
au moins un orifice d'alimentation de liquide de lavage dans la surface du corps de prothèse de fémur qui est relié à l'intérieur du corps de prothèse de fémur avec l'orifice de sortie de liquide de lavage du corps de prothèse de fémur en laissant passer le liquide, où
l'au moins un orifice d'évacuation de liquide de lavage du corps de prothèse de fémur et l'au moins un orifice d'alimentation de liquide de lavage du corps de prothèse de fémur sont disposés à l'extérieur de l'au moins une surface de fixation (42, 92) du corps de prothèse de fémur, et où
l'au moins un orifice d'évacuation de liquide de lavage du corps de prothèse de fémur et l'au moins un orifice d'alimentation de liquide de lavage du corps de prothèse de fémur sont disposés à l'extérieur des condyles (43, 44, 93, 94) du composant fémoral (2, 52).

15. Système d'espaceur de genou selon l'une des revendications précédentes, **caractérisé en ce**
**qu'**au moins un orifice d'évacuation de liquide de lavage (10, 22, 60, 72) parmi l'au moins un orifice d'évacuation de liquide de lavage (10, 22, 60, 72) ou au moins un orifice d'alimentation de liquide de lavage (12, 24, 62, 74) parmi l'au moins un orifice d'alimentation de liquide de lavage (12, 24, 62, 74) est disposé au niveau d'un côté distal d'une extension (26, 76) s'étendant en direction distale entre deux surfaces de fixation (5, 6, 55, 56) du composant tibial (1, 51).
